# FASCICULE DE BREVET EUROPEEN

(11) **EP 1 712 643 B1**
(45) Date de publication et mention de la délivrance du brevet: **06.04.2016**
(21) Numéro de dépôt: 06290592.2
(22) Date de dépôt: 12.04.2006
(51) Int. Cl.: C12Q 1/68

(54) **Prévention des toxicités dues au 5-fluorouracile**
Verhinderung der durch 5-Fluorouracil verursachten Toxizitäten
Preventition of toxicities due to 5-fluorouracil

(30) Priorité: 12.04.2005 FR 0503616
(43) Date de publication de la demande: 18.10.2006
(73) Titulaire: Institut de Cancérologie de l'Ouest (I.C.O), 49933 Angers Cedex 9 (FR); UNIVERSITE D'ANGERS, 49000 Angers (FR)
(72) Inventeur: Gamelin, Erick, 49080 Bouchemaine (FR); Morel, Alain, 49610 Juigne sur Loire (FR); Boisdron-Celle, Michèle, 49170 Saint Leger des Bois (FR)
(74) Mandataire: Regimbeau

(56) Documents cités:
- WO-A-97/35034
- WO-A-03/018837
- GAMELIN E ET AL: "Correlation between uracil and dihydrouracil plasma ratio, fluorouracil (5-FU) pharmacokinetic parameters, and tolerance in patients with advanced colorectal cancer: A potential interest for predicting 5-FU toxicity and determining optimal 5-FU dosage" JOURNAL OF CLINICAL ONCOLOGY, vol. 17, no. 4, avril 1999 (1999-04), pages 1105-1110, XP002395547 ISSN: 0732-183X
- JIANG HAO ET AL: "Important role of the dihydrouracil/uracil ratio in marked interpatient variations of fluoropyrimidine pharmacokinetics and pharmacodynamics" JOURNAL OF CLINICAL PHARMACOLOGY, vol. 44, no. 11, novembre 2004 (2004-11), pages 1260-1272, XP009071315 ISSN: 0091-2700
- VAN KUILENBURG A B P ET AL: "Clinical implications of dihydropyrimidine dehydrogenase (DPD) deficiency in patients with severe 5-fluorouracil-associated toxicity: Identification of new mutations in the DPD gene" CLINICAL CANCER RESEARCH 2000 UNITED STATES, vol. 6, no. 12, 2000, pages 4705-4712, XP002360415 ISSN: 1078-0432
- GARG M B ET AL: "Simple liquid chromatographic method for the determination of uracil and dihydrouracil plasma levels: a potential pretreatment predictor of 5-fluorouracil toxicity" JOURNAL OF CHROMATOGRAPHY B: BIOMEDICAL SCIENCES & APPLICATIONS, ELSEVIER, AMSTERDAM, NL, vol. 774, no. 2, 15 juillet 2002 (2002-07-15), pages 223-230, XP004365818 ISSN: 1570-0232
- CICCOLINI J ET AL: "A simple and rapid high-performance liquid chromatographic (HPLC) method for 5-fluorouracil (5-FU) assay in plasma and possible detection of patients with impaired dihydropyrimidine dehydrogenase (DPD) activity" JOURNAL OF CLINICAL PHARMACY AND THERAPEUTICS 2004 UNITED KINGDOM, vol. 29, no. 4, 2004, pages 307-315, XP002360414 ISSN: 0269-4727
- MATTISON L K ET AL: "Implications of dihydropyrimidine dehydrogenase on 5-fluorouracil pharmacogenetics and pharmacogenomics" PHARMACOGENOMICS 2002 UNITED KINGDOM, vol. 3, no. 4, 2002, pages 485-492, XP008057741 ISSN: 1462-2416
- AHLUWALIA R ET AL: "Use of pyrosequencing to detect clinically relevant polymorphisms in dihydropyrimidine dehydrogenase" CLINICAL CHEMISTRY 01 OCT 2003 UNITED STATES, vol. 49, no. 10, 1 octobre 2003 (2003-10-01), pages 1661-1664, XP002360413 ISSN: 0009-9147
- BOISDRON-CELLE M A ET AL: "FLUOROPYRIMIDINE-RELATED SEVERE TOXICITY. COMPARISON OF DIFFERENT METHODS FOR DETECTING DIHYDROPYRIMIDINE DEHYDROGENASE (DPD) DEFICIENCY AND PREDICTING 5-FU METABOLISM" PROCEEDINGS OF THE ANNUAL MEETING OF THE AMERICAN ASSOCIATION FOR CANCER RESEARCH, NEW YORK, NY, US, vol. 43, mars 2003 (2003-03), page 321,AB1593, XP001128790 ISSN: 0197-016X
- MILANO G ET AL: "Can dihydropyrimidine dehydrogenase impact 5-fluorouracil-based treatment?" EUROPEAN JOURNAL OF CANCER 2000 UNITED KINGDOM, vol. 36, no. 1, 2000, pages 37-42, XP002360416 ISSN: 0959-8049

## Description

La présente invention concerne une méthode de diagnostic in vitro d'une sensibilité accrue au 5-fluorouracile, ladite méthode comprenant la réalisation sur un ou plusieurs prélèvements, dont au moins un prélèvement sanguin, de la combinaison d'au moins deux tests in vitro. Elle concerne également une méthode de diagnostic *in vitro* de la prédisposition d'un sujet au développement d'une toxicité à la suite d'un traitement comprenant du 5-fluorouracile et/ou ses prodrogues. Elle concerne en outre des kits permettant la mise en oeuvre desdites méthodes.

Les cancers colorectaux, tumeurs solides, représentent avec les cancers du sein et du poumon l'une des premières causes de mortalité par cancers. En effet, ils sont la cause de 16 000 décès par an en France. En outre, chaque année, environ un million de nouveaux cas de cancer colorectal sont diagnostiqués dans le monde (environ 150000 aux Etats-Unis et 36000 en France), dont la moitié des patients présenteront une forme métastatique. Ceci pose donc un véritable problème de santé publique dans les pays occidentaux.

L'un des agents anti-cancéreux les plus utilisés depuis plusieurs années dans le cadre du traitement de ces pathologies est le 5-fluorouracile (5-FU). Ce médicament entre dans la composition de plus de 50% des protocoles de chimiothérapie et est utilisé dans les cancers métastatiques mais aussi en situation adjuvante, c'est à dire chez des patients potentiellement guéris.

Le 5-FU, un antimétabolite de la famille des fluoropyrimidines, est un analogue des bases pyrimidiques. Son mécanisme de cytotoxicité implique sa conversion en nucléotides actifs (voie anabolique), qui vont bloquer la synthèse d'acide désoxyribonucléique (ADN). Cependant, il existe un équilibre entre l'activation enzymatique du 5-FU et son élimination (voie catabolique). L'enzyme initiale et limitante du catabolisme de cet anti-cancéreux est la Dihydropyrimidine Deshydrogénase (DPD). Cette enzyme ubiquitaire est un facteur majeur de la biodisponibilité du 5-FU puisque plus de 80 % de l'agent anti-cancéreux administrés sont catabolisés par cette dernière en 5 dihydrofluorouracile. Seuls 20% du 5-FU administré est donc normalement disponible pour la voie anabolique nécessaire à son action cytotoxique.

Cependant, l'activité de cette enzyme est soumise à un polymorphisme génétique, et à ce jour, il a été découvert plus de 30 mutations sur le gène codant pour la DPD (situé sur le chromosome 1 p22), dont certaines entraînent un déficit enzymatique.

Or, il a été montré qu'un déficit partiel ou total en DPD engendre chez des patients traités par du 5-FU des toxicités souvent sévères, voire mortelles. La plupart des épisodes de toxicité dus au 5-FU surviennent principalement au niveau des tissus à renouvellement rapides, comme la moelle osseuse (toxicité hématologique : neutropénie, thrombopénie, anémie), les muqueuses de l'appareil digestif (nausées et vomissements, mucites, ulcérations de la muqueuse oropharyngée, diarrhées sévères) et les cellules de la peau (alopécies, dermatites, érythrodermie palmo-plantaire ou syndrome «mains - pieds») (Diasio R.B., et al. 1988. J. Clin. Invest. 81, 47-51). Une toxicité oculaire ou neurologique peut également apparaître. Les différentes toxicités sont échelonnées selon différents grades numérotés de 0 à IV définis par l'OMS. Les toxicités graves au 5-FU ne sont pas rares. Une étude récente portant sur l'analyse rétrospective de 1219 patients atteints de cancers colorectaux traités par du 5-FU montre que 31 à 34 % de ces patients ont présenté une toxicité de grade III à IV, et 0,5 % une toxicité mortelle (Van Kuilenburg et al. 2004. European Journal of Cancer 40, 939-950).

Il serait donc très utile de disposer d'une méthode permettant de diagnostiquer à partir d'un échantillon biologique d'un patient si celui-ci possède un déficit partiel ou total en DPD engendrant chez ce patient une sensibilité accrue au 5-FU et donc une prédisposition au développement d'une toxicité suite à un traitement comprenant du 5-FU. Une telle méthode permettrait alors d'adapter la dose de 5-FU à administrer à chaque patient.

Différents paramètres ont été décrits comme des indicateurs de l'activité enzymatique de la DPD. En particulier, la concentration en uracile plasmatique a été rapportée comme pouvant être élevée dans les déficits en DPD. Cependant, aucune véritable corrélation n'a été rapportée, et aucune valeur seuil permettant de déterminer la présence ou l'absence d'un risque de toxicité n'a été déterminée à ce jour. De même, le rapport des concentrations plasmatiques en dihydrouracile et en uracile (rapport UH2/U) a été corrélé avec la clairance plasmatique du 5-FU, qui est elle-même corrélée avec le risque de développer une toxicité. Cependant, aucune valeur seuil n'a encore été rapportée. Enfin, certaines mutations dans le gène codant pour la DPD ont été décrites chez des patients traités au 5-FU et ayant présenté des toxicités importantes. Jusqu'à présent, 39 mutations dont certaines ont été corrélées à un déficit en DPD, ont été répertoriées dans la phase codante et au niveau du promoteur de ce gène (Van Kuilenburg et al. 2004. European Journal of Cancer 40, 939-950). Il s'agit principalement de substitutions et de délétions. Cependant, aucune étude de grande envergure n'a réellement permis de déterminer parmi toutes ces mutations décrites quelles sont celles réellement corrélées avec une sensibilité accrue au 5-FU, et d'estimer quelle est leur fréquence dans la population.

Gamelin et al. (J. Clin. Oncol., 17(4): 1105-1110, 1999) décrit l'existence d'une corrélation entre le rapport [UH2] / [U] et la toxicité due au 5-FU. Garg et al. (J Chromatogr B Biomed Sci Appl, 774(2) : 223-230, 2002) décrit une méthode pour mesurer par HPLC les concentrations plasmatiques en uracile et dihydrouracile. WO 03/18837 divulgue une méthode et des kits pour déterminer la compatibilité médicamenteuse d'un sujet par la recherche de mutations dans des gènes d'enzymes impliquées dans le métabolisme de détoxification. Ciccolini et al. (J Clin Pharm Ther., 29(4): 307-15, 2004) décrit une méthode pour mesurer par HPLC la valeur du rapport [UH2] / [U]. WO 97/35034 décrit la caractérisation de la mutation IVS14+1G>A du gène de la DPD. Jiang Hao et al. (J Clin Pharmacol., 44(11):1260-72, 2004) décrit l'existence d'une corrélation entre le rapport [UH2] / [U] et des données physiologiques après administration du 5-FU. Van Kuilenburg et al. (Clin. Cancer Res., 6(12): 4705-4712, 2000) décrit le séquençage du gène de la DPD. Mattison et al. (Pharmacogenomics., 3(4):485-92, 2002) et Milano et al. (Eur. J. Cancer, 36(1) : 37-42, 2000) sont des revues concernant le rôle de la DPD dans les toxicités induites par le 5-FU. Ahluwalia et al. (Clin. Chem., 49(10) : 1661-1664, 2003) décrit l'utilisation de la pyroséquençage pour déterminer la présence de mutations dans le gène de la DPD.

Les inventeurs ont réalisé pour la première fois une étude de grande envergure afin de déterminer la pertinence des différents paramètres décrits ci-dessus (concentration plasmatique en uracile, rapport UH2/U, polymorphisme génétique) vis-à-vis d'un diagnostic d'une sensibilité accrue au 5-FU. Leurs résultats montrent de façon surprenante que, bien qu'aucun de ces paramètres ne permette seul d'établir un diagnostic suffisamment sensible et spécifique, la combinaison d'au moins deux de ces paramètres, pour lesquels des valeurs seuils ont été déterminées, permet en revanche un diagnostic à la fois sensible et spécifique de la présence ou de l'absence d'une sensibilité accrue au 5-FU, et donc d'une prédisposition au développement de toxicités suite à un traitement comprenant du 5-FU.

La présente invention a pour objet une méthode de diagnostic in vitro de la prédisposition d'un patient au développement d'une toxicité à la suite d'un traitement comprenant du 5-fluorouracile telle que celle de la revendication 1.

Il est ici décrit une méthode de diagnostic in vitro d'une sensibilité accrue au 5-fluorouracile, ladite méthode comprenant la réalisation sur un ou plusieurs échantillons biologiques, dont au moins un prélèvement sanguin, de la combinaison d'au moins deux tests in vitro choisis dans le groupe constitué de :
- la détermination de la présence ou de l'absence d'une mutation dans le gène codant pour la Dihydropyrimidine Déshydrogénase,
   - la mesure de la concentration en uracile plasmatique, et
   - la mesure du rapport des concentrations plasmatiques dihydrouracile/uracile.

Par "sensibilité accrue" d'un sujet au 5-fluorouracile, on entend toute augmentation par rapport à un sujet contrôle de la proportion de 5-FU métabolisée par la voie anabolique. Chez un "sujet contrôle", 20% du 5-FU administré sont métabolisés par la voie anabolique. De manière avantageuse, chez un sujet présentant une sensibilité accrue au 5-FU, au moins 40%, au moins 60%, au moins 80%, au moins 90%, ou au moins 95% du 5-FU administré sont métabolisés par la voie anabolique.

Par "échantillon biologique", on entend tout échantillon prélevé sur un sujet, par exemple un prélèvement sanguin, un prélèvement d'organe (biopsie par exemple), un prélèvement de moelle osseuse, etc. Pour la mesure de la concentration en uracile plasmatique ou du rapport des concentrations plasmatiques dihydrouracile/ uracile, l'échantillon est de préférence un prélèvement sanguin ou un échantillon de plasma. Pour la détermination de la présence ou de l'absence d'une mutation dans le gène codant pour la Dihydropyrimidine Déshydrogénase, l'échantillon biologique peut être tout type d'échantillon biologique provenant dudit sujet contenant des cellules nucléées, par exemple un prélèvement sanguin, un prélèvement d'organe (par exemple des cellules isolées à partir d'un ganglion potentiellement métastasé retiré au patient). De préférence, l'échantillon biologique est un prélèvement sanguin.

Une "mutation" dans un gène signifie ici toute modification dans la séquence nucléique dudit gène. Une telle modification peut notamment être choisie dans le groupe comprenant les substitutions, aussi bien les transversions que les transitions, les délétions et les insertions.

La présente description concerne également une méthode de diagnostic *in vitro* de la prédisposition d'un sujet au développement d'une toxicité à la suite d'un traitement comprenant du 5-fluorouracile, ladite méthode comprenant la réalisation sur un ou plusieurs échantillons biologiques, dont au moins un prélèvement sanguin, de la combinaison d'au moins 2 tests in vitro choisis dans le groupe constitué de :
- la détermination de la présence ou de l'absence d'une mutation dans le gène codant pour la Dihydropyrimidine Déshydrogénase,
   - la mesure de la concentration en uracile plasmatique, et
   - la mesure du rapport des concentrations plasmatiques dihydrouracile/uracile.

Par "prédisposition", on entend la tendance générale d'un sujet à être, plus particulièrement que d'autres, affecté par tel ou tel syndrome. Un sujet prédisposé aura une probabilité plus forte de développer une toxicité à la suite d'un traitement comprenant du 5-fluorouracile. Un sujet non prédisposé possède une probabilité de l'ordre de 6% de développer une toxicité précoce aiguë sévère dès la première administration de 5-fluorouracile. De préférence, la probabilité de développer une toxicité d'un individu prédisposé est au moins 5 fois, de préférence au moins 10 fois, voire au moins 15 fois supérieure à celle d'un individu non prédisposé. La probabilité de développer une toxicité à la suite d'un traitement comprenant du 5-fluorouracile d'un patient prédisposé est donc de manière avantageuse d'au moins 30 %, au moins 60%, voire au moins 90%.

Par "développement d'une toxicité", on entend la survenue de troubles physiologiques indésirables, gênants et/ou dangereux, induits par l'administration d'un traitement comprenant du 5-fluorouracile. La toxicité peut notamment consister en une toxicité hématologique (neutropénie, thrombopénie, anémie), digestive (nausées et vomissements, mucites, ulcérations de la muqueuse oropharyngée, diarrhées sévères), cutanéo-muqueuse (alopécies et des dermatites, érythrodermie palmo-plantaire ou syndrome «mains - pieds»), oculaire ou neurologique. Selon leur sévérité et leur durée, les toxicités dues au 5-fluorouracile peuvent être hiérarchisées selon quatre grades définis par l'OMS.

Dans un mode de réalisation avantageux, une des méthodes décrites ci-dessus comprend la combinaison de la détermination de la présence ou de l'absence d'une mutation dans le gène codant pour la Dihydropyrimidine Déshydrogénase et de la mesure de la concentration en uracile plasmatique. De manière encore plus avantageuse, ladite méthode comprend la combinaison de la détermination de la présence ou de l'absence d'une mutation dans le gène codant pour la Dihydropyrimidine Déshydrogénase, de la mesure de la concentration en uracile plasmatique, et de la mesure du rapport des concentrations plasmatiques dihydrouracile/uracile.

La présente description concerne également une méthode d'évaluation in vitro à partir d'un ou plusieurs échantillons biologiques, dont au moins un prélèvement sanguin, de la dose de 5-fluorouracile adaptée à un patient atteint d'une maladie qui peut être traitée avec du 5-fluorouracile, de préférence un cancer, ladite méthode comprenant les étapes suivantes :
(a) la collecte des résultats de la détermination de la présence ou de l'absence d'une mutation dans le gène codant pour la Dihydropyrimidine Déshydrogénase, de la mesure de la concentration en uracile plasmatique, et éventuellement de la mesure du rapport des concentrations plasmatiques dihydrouracile/uracile; et
(b) la détermination de la dose de 5-fluorouracile adaptée au patient en fonction des résultats obtenus à l'étape (a).

Ladite méthode d'évaluation in vitro à partir d'un ou plusieurs échantillons biologiques, dont au moins un prélèvement sanguin, de la dose de 5-fluorouracile adaptée à un patient atteint d'une maladie qui peut être traitée avec du 5-fluorouracile peut en outre comprendre les étapes suivantes :
(c) la mesure à partir d'au moins un prélèvement sanguin de la concentration plasmatique en 5-fluorouracile en fin d'injection et au cours des heures et/ou jours suivant l'injection de 5-fluorouracile, et
(d) la détermination de la dose suivante de 5-fluorouracile adaptée au patient en fonction du résultat de l'étape (c).

Une dose de 5-fluorouracile "adaptée à un patient" est définie comme une dose de 5-fluorouracile suffisamment forte pour obtenir l'effet thérapeutique désiré, par exemple une cytotoxicité vis-à-vis de cellules tumorales, et suffisamment faible pour ne pas provoquer le développement d'une toxicité de grade supérieur ou égal à III. De préférence, une dose adaptée ne provoque pas de toxicité de grade supérieur ou égal à II.

Le diagnostic in vitro d'une sensibilité accrue au 5-fluorouracile, de la prédisposition d'un sujet au développement d'une toxicité à la suite d'un traitement comprenant du 5-fluorouracile, et/ou la détermination de la dose de 5-fluorouracile adaptée au patient sont réalisés par comparaison des résultats des au moins 2 tests in vitro réalisés avec une liste de mutations significatives du gène codant pour la Dihydropyrimidine Déshydrogénase et des valeurs de référence ou valeurs seuils pour la concentration en uracile plasmatique et pour le rapport des concentrations plasmatiques dihydrouracile/uracile.

Une « mutation significative » dans le gène codant pour la DPD est définie comme une mutation dont la présence génère une modification de l'activité enzymatique de la DPD, de préférence une diminution de son activité enzymatique. De préférence, une "mutation significative" dans le gène codant pour la DPD entraîne une diminution d'au moins 50%, au moins 60%, au moins 75%, au moins 80% ou au moins 90% de l'activité enzymatique de la DPD. De manière avantageuse, la liste des mutations significatives du gène codant pour la DPD comprend les mutations I560S, IVS14+1G>A, D949V, L155Stop, R21Stop, Del TCAT295, Del T812, Del TG1039, E386Stop, et Del C1897. De manière également avantageuse, une valeur seuil pour la concentration en uracile plasmatique est 15 µg/L, et des valeurs seuils pour le rapport des concentrations plasmatiques dihydrouracile/uracile sont 1 ; 3 ; et/ou 6.

Dans un mode de réalisation avantageux, le diagnostic in vitro d'une sensibilité accrue au 5-fluorouracile, de la prédisposition d'un sujet au développement d'une toxicité à la suite d'un traitement comprenant du 5-fluorouracile, et/ou la détermination de la dose de 5-fluorouracile adaptée au patient est réalisé grâce à un algorithme de décision prenant en compte les résultats obtenus pour les au moins 2 tests in vitro réalisés.

L'invention a ainsi pour objet une méthode telle que celle de la revendication 3, qui comprend une étape supplémentaire de détermination de la dose de 5-fluorouracile à administrer au patient, dans laquelle la dose est déterminée à l'aide d'un algorithme de décision.

Un "algorithme de décision" est défini comme un ensemble d'instructions permettant, à partir d'un jeu de données connues, et uniquement par le suivi automatique de ces instructions, de déterminer la conduite à tenir pour ledit jeu de données particulier. Un tel algorithme peut se présenter soit sous la forme d'une liste d'instructions dépendantes et/ou indépendantes aboutissant à une décision recommandée en fonction du jeu de données, soit sous la forme d'un programme informatique qui, à partir de l'entrée du jeu de données, retourne la décision fournie par l'algorithme.

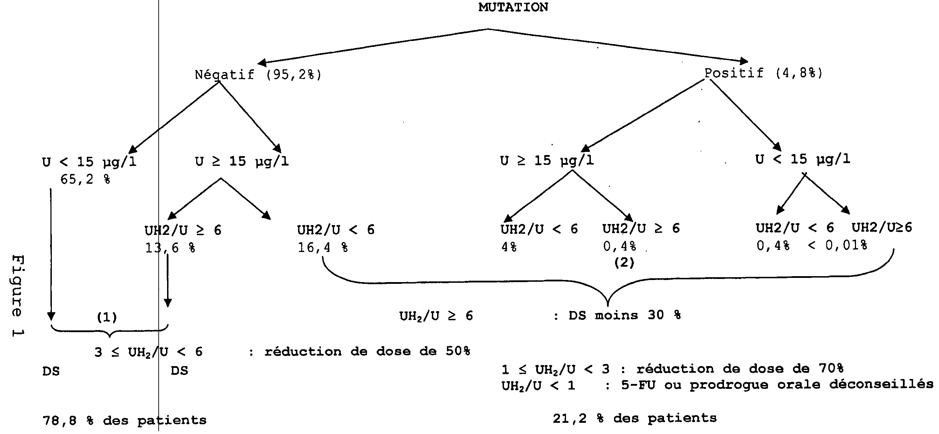

De préférence, l'algorithme de décision comprend les étapes suivantes (voir Figure 1) :
(a) En cas
   - d'absence de mutation dans le gène codant pour la Dihydropyrimidine Déshydrogénase parmi celles recherchées et si la concentration en uracile plasmatique est inférieure à 15 µg/L, ou
   - d'absence de mutation dans le gène codant pour la Dihydropyrimidine Déshydrogénase parmi celles recherchées et si la concentration en uracile plasmatique est supérieure à 15 µg/l mais que le rapport UH₂/U est supérieur ou égal à 6,
le sujet ne possède pas de sensibilité accrue au 5-fluorouracile, n'est pas prédisposé au développement d'une toxicité à la suite d'un traitement comprenant du 5-fluorouracile, et le traitement peut avoir lieu aux doses standard de 5-fluorouracile,
(b) Dans tous les autres cas, c'est-à-dire, en cas :
   - d'absence de mutation dans le gène codant pour la Dihydropyrimidine Déshydrogénase parmi celles recherchées, si la concentration en uracile plasmatique est supérieure ou égale à 15 µg/L, et UH₂/U inférieur à 6,
   - de présence de mutation significative hétérozygote ou homozygote dans le gène codant pour la Dihydropyrimidine Déshydrogénase, sans augmentation de la concentration en uracile plasmatique,
   - de présence de mutation significative hétérozygote ou homozygote dans le gène codant pour la Dihydropyrimidine Déshydrogénase, et d'augmentation de la concentration en uracile plasmatique,
   le sujet possède une sensibilité accrue au 5-fluorouracile, est prédisposé au développement d'une toxicité à la suite d'un traitement comprenant du 5-fluorouracile, et le rapport des concentrations plasmatiques dihydrouracile/uracile est calculé pour confirmer le déficit et déterminer la dose de 5-FU à administrer. Si celui-ci est:
   (i) supérieur à 6 : la dose initiale de 5-fluorouracile est diminuée de 30% par rapport à la dose standard.
   (ii) compris entre 3 et 6 : la dose initiale de 5-fluorouracile est réduite de 50% par rapport à la dose standard et une mesure de la concentration plasmatique en 5-fluorouracile au cours des jours/heures suivant l'injection d'une dose de 5-fluorouracile est réalisée.
   (iii) compris entre 1 et 3 : la dose initiale de 5-fluorouracile est réduite de 70% par rapport à la dose standard et une mesure de la concentration plasmatique en 5-fluorouracile au cours des jours/heures suivant l'injection d'une dose de 5-fluorouracile est réalisée
   (iv) inférieur à 1 : le traitement par le 5-fluorouracile est déconseillé, même à doses réduites. Si le traitement doit malgré tout être mis en oeuvre, la dose initiale est réduite de 80% et une mesure de la concentration plasmatique en 5-fluorouracile au cours des jours/heures suivant l'injection d'une dose de 5-fluorouracile est indispensable.

De plus, en cas de mutation significative homozygote du gène codant pour la Dihydropyrimidine Déshydrogénase, d'une concentration en uracile plasmatique supérieure à 100 µg/L, et/ou d'un rapport dihydrouracile/uracile inférieur à 1, des précautions particulières de surveillance sont mises en place, même à doses réduites.

Une « dose standard » de 5-fluorouracite est définie comme étant la dose recommandée dans le protocole en fonction de la localisation, une telle dose recommandée étant connue de l'homme du métier. Dans les cancers colorectaux, différents protocoles sont couramment utilisés :
- protocole LV5FU2 standard : IV bolus de 5-FU à 400mg/m², puis une perfusion de 22 heures de 5-FU à 600 mg/m² à J1 puis J2 tous les 15 jours.
- protocole LV5FU2 simplifié : IV bolus de 5-FU à 400 mg/m² à J1, puis une perfusion continue de 46 heures de 5-FU à 2400 mg/m² tous les 15 jours.
- protocole 5-FU hebdomadaire : 5-FU à 1200, 1800, ou 2600 mg/m² en perfusion de 4 heures, 8 heures ou 24 heures respectivement.

Dans les cancers ORL, oesophagien ou en cas d'association avec une radio-chimiothérapie, un protocole couramment utilisé est : 5-FU à une dose de 800 à 1000 mg/m² en perfusion continue de 24 heures pendant 4 à 5 jours toutes les 3 semaines.

Dans les cancers du sein, un protocole courant est : IV bolus de 5-FU à 500 mg/m² toutes les 3 semaines.

Un traitement est dit « déconseillé » lorsque la toxicité qu'il peut induire met en jeu le pronostic vital du patient avec une probabilité supérieure à 90%.

Dans un mode de réalisation avantageux des méthodes de diagnostic et/ou d'évaluation de dose adaptée in vitro, la mutation dans le gène de la Dihydropyrimidine Déshydrogénase est choisie dans le groupe comprenant :
- I560S,
- IVS14+1G>A,
- D949V,
- L155Stop,
- R21Stop,
- Del TCAT295,
- Del T812,
- Del TG1039,
- E386Stop, et
- Del C1897.

Les caractéristiques précises de ces mutations sont décrites dans le Tableau 1 qui suit.

**Tableau 1. Caractéristiques des mutations I560S, IVS14+1G>A, D949V, L155Stop, R21Stop, Del TCAT295, Del T812, Del TG1039, E386Stop, et Del C1897.**

| Mutation | **Position dans le gène** | **Conséquence au niveau du gène** | **Conséquence au niveau de la protéine** |
|---|---|---|---|
| R21 Stop (=C61T) | exon 2 | Substitution d'une cytosine par une thymine en position 61 | Protéine tronquée avant les principaux sites de liaison aux co-enzymes et au substrat, pas d'activité |
| Del TCAT295 | exon 4 | Délétion de 4 bases en position 295 | Codon stop prématuré, pas d'activité |
| L155Stop (=T464A) | exon 5 | Substitution d'une thymine par une adénine en position 464 | Codon stop en position 155, protéine tronquée, inactive |
| Del T812 | exon 8 | Délétion d'une thymine en position 812 | Codon stop prématuré, pas d'activité |
| Del TG1039 | exon 10 | Délétion de 4 bases en position 1039 | Codon stop prématuré, pas d'activité |
| E386Stop (=G1156T) | exon 11, codon 386 | Substitution d'une guanine par une thymine en position 1156 | Codon stop prématuré, pas d'activité |
| I560S (=T1679G) | exon 13, | Substitution d'une thymine par une guanine en position 1679 | Faux sens, site FAD, interférence avec la fixation du cofacteur, pas d'activité |
| Del C1897 | exon 14 | Délétion d'une cytosine en position 1897 | Mutation nonsens, codon stop avant le site de fixation du substrat |
| IVS14+1G>A | intron 14 | Substitution d'une guanine par une adénine en début d'intron | délétion complète de l'exon 14 lors de l'épissage (perte de la zone de fixation du substrat) |
| D949V (=A2846T) | exon 22 | Substitution d'une adénine par une thymine en position 2846 | Mutation faux sens, interférence directe avec la fixation du cofacteur ou le transport d'électrons, fonction [4Fe-4S] altérée |

Dans un mode de réalisation avantageux, le protocole de détermination à partir des cellules d'un échantillon provenant du sujet de la présence ou de l'absence d'une mutation dans le gène codant pour la Dihydropyrimidine Déshydrogénase comporte les étapes suivantes:
- l'extraction de l'ADN génomique, ou l'extraction de l'ARN total et sa rétrotranscription en ADN complémentaire,
- l'amplification en parallèle ou en multiplex d'un ou plusieurs fragments du gène codant pour la Dihydropyrimidine Déshydrogénase, et
- la détermination de la taille du produit d'amplification, où la taille du produit d'amplification indique la présence ou l'absence d'une mutation dans le gène codant pour la Dihydropyrimidine Déshydrogénase.

Dans un autre mode de réalisation avantageux, le protocole de détermination à partir des cellules d'un échantillon provenant du sujet de la présence ou de l'absence d'une mutation dans le gène codant pour la Dihydropyrimidine Déshydrogénase comporte les étapes suivantes:
- l'extraction de l'ADN génomique,
- l'amplification en parallèle ou en multiplex d'un ou plusieurs fragments du gène codant pour la Dihydropyrimidine Déshydrogénase, et
- le séquençage des fragments amplifiés permettant l'identification de la présence ou de l'absence d'une variation dans le gène codant pour la Dihydropyrimidine Déshydrogénase.

Dans un autre mode de réalisation encore plus avantageux, le protocole de détermination de la présence ou de l'absence d'une mutation dans le gène codant pour la Dihydropyrimidine Déshydrogénase est une combinaison des deux protocoles précédemment décrits.

L'extraction à partir des cellules d'un échantillon du sujet de l'ADN génomique ou de l'ARN total peut être réalisée par toutes techniques connues de l'homme du métier, ou par l'utilisation de tout kit d'extraction d'ADN génomique ou d'ARN total disponible dans le commerce. L'ARN peut être rétrotranscrit en ADN complémentaire (ADNc) par toute technique connue de l'homme du métier. De même, l'amplification des fragments d'intérêt peut être réalisée par toute technique décrite dans la littérature scientifique, notamment, l'amplification sera réalisée par PCR. Quand plusieurs fragments sont amplifiés simultanément, l'amplification peut être réalisée soit "en parallèle", c'est-à-dire que les différents fragments sont amplifiés simultanément mais dans des réactions distinctes, soit en « multiplex », c'est-à-dire que les différents fragments sont amplifiés simultanément dans une même réaction. La détermination de la taille des produits amplifiés peut être réalisée par toute technique connue de l'homme du métier, notamment par électrophorèse en présence de marqueurs de taille, la révélation des produits de PCR étant réalisée par tout moyen connu de l'homme du métier, notamment l'utilisation d'un agent intercalant de l'ADN.

Le séquençage des fragments amplifiés peut aussi être réalisé par toute technique connue de l'homme du métier. De manière avantageuse, la technique de séquençage utilisée est le pyroséquençage (ou mini-séquençage luminométrique).

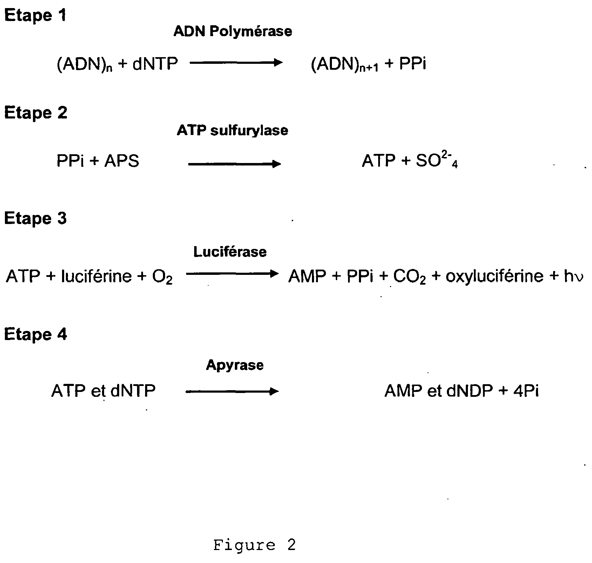

Le pyroséquençage est une méthode de séquençage par détection de l'incorporation des nucléotides sur une matrice spécifique. Elle est basée sur un système de cascade enzymatique utilisant quatre enzymes (ADN polymérase, ATP sulfurylase, luciférase et apyrase) et deux substrats spécifiques (APS, luciférine). Le principe de cette technique ainsi que différentes améliorations du protocole de base ont été publiées (Ronaghi M, et al. 1996. Anal Biochem 242: 84-89; Ronaghi M. 2001. Genome Research, 3-11.) et les différentes étapes de cette technique sont détaillées sur la Figure 2 : après amplification et dénaturation du fragment d'intérêt, un des deux brins est hybridé à une amorce de séquence et mis en présence du milieu réactionnel comprenant les quatre enzymes et les substrats. Pour l'analyse de chaque nucléotide, quatre étapes sont nécessaires pour obtenir une succession de pics appelée un pyrogramme™. La lecture de ce pyrogramme permet ensuite de déterminer la séquence nucléotidique obtenue et d'identifier le génotype.

Une amélioration du protocole consiste à employer dans les dNTP du déoxyadénosine alpha-thio triphosphate (dATPaS) à la place du dATP naturel car il peut être utilisé par l'ADN polymérase, mais n'est pas reconnu par la luciférase. Ainsi, même si la dégradation par l'apyrase est incomplète, seul le signal provenant de l'ATP généré à l'étape 2 (Figure 2) est quantifié par l'ATP sulfurylase.

Cette technique permet non seulement la détection de mutations telles que substitutions, délétions et insertions, mais également le séquençage de courts fragments (maximum 70 nucléotides). Elle cumule les avantages d'être rapide, reproductible, peu onéreuse et facilement adaptable à tout type de mutation. L'intérêt du pyroséquençage réside également dans sa compatibilité avec une utilisation en routine. 500µL de sang total sont suffisants pour extraire l'ADN leucocytaire servant au génotypage. Après amplification par PCR et une étape de dénaturation et de lavage des fragments d'intérêt, l'analyse d'une plaque contenant 96 échantillons est effectuée en 20 min.

D'autres modifications peuvent être apportées au protocole pour améliorer la sensibilité et la fiabilité de cette technique. Dans un mode de réalisation avantageux, au moins une des amorces de séquences utilisées pour le pyroséquençage contient un ou plusieurs nucléotides modifiés en ce qu'ils contiennent un pont méthylène 2'O-4'C les maintenant dans une conformation bicyclique rigide. La présence d'un tel nucléotide induit la base ADN adjacente à adopter la même conformation, ce qui aboutit à une structure thermodynamiquement plus stable (Singh SK, et al. 1998. J. Org. Chem. 63: 6078-6079; Nielsen KE, et al. 2000. Bioconjug. Chem. 11: 228-238). Une amorce de pyroséquençage peut comporter 100% de nucléotides modifiés ou alors un mélange de nucléotides modifiés et non modifiés.

De manière avantageuse, les amorces utilisées pour l'amplification par PCR sont choisies dans le groupe constitué de SEQ ID NO : 3, 4, 9, 10, 15 à 20, 23 à 28, 31, 32, 37 et 38.

De même, les amorces utilisées pour le pyroséquençage des fragments d'intérêt sont de préférence choisies dans le groupe constitué de SEQ ID NO : 40, 43, 47, 49, 50, 53 à 55, 57 et 60.

Dans l'ensemble des méthodes décrites précédemment, la mesure de la concentration en uracile et/ou en dihydrouracile plasmatique peut être réalisée par toute technique connue de l'homme du métier. De manière avantageuse, la mesure de la concentration en uracile et/ou en dihydrouracile plasmatique est réalisée par chromatographie liquide haute performance avec- détection UV, en utilisant une colonne possédant une phase stationnaire composée de particules de carbone sphériques totalement poreuses de type Hypercarb™ fournie par Thermo Electron (Courtaboeuf, France).

Chacune des méthodes décrites précédemment est de manière avantageuse mise en oeuvre sur un ou plusieurs échantillons biologiques, dont au moins un prélèvement sanguin, d'un patient atteint d'une maladie qui peut être traitée avec du 5-fluorouracile.

Les maladies qui peuvent être traitées avec du 5-fluorouracile comprennent notamment les cancers, plus particulièrement le cancer du sein, les cancers ORL, le cancer de l'ovaire, de la prostate, du pancréas, de l'estomac, du rein, du colon, du rectum, les tumeurs carcinoïdes, les kératoses solaires malignes, la maladie de Bowen, le condylome acumine, et le cholangiocarcinome.

La présente description concerne également un kit de diagnostic in vitro d'une sensibilité accrue au 5-fluorouracile ou d'une prédisposition d'un sujet au développement d'une toxicité à la suite d'un traitement comprenant du 5-fluorouracile, ledit kit comprenant au moins deux réactifs choisis dans le groupe constitué de :
- réactifs permettant la mesure de la concentration en uracile plasmatique,
- réactifs permettant la mesure du rapport des concentrations plasmatiques dihydrouracile/uracile, et
- réactifs permettant la détermination de la présence ou l'absence d'une mutation dans le gène codant pour la Dihydropyrimidine Déshydrogénase.

La présente description concerne aussi un kit de diagnostic in vitro permettant la détermination de la dose de 5-fluorouracile adaptée à un patient atteint d'une maladie qui peut être traitée avec du 5-fluorouracile, de préférence un cancer, ledit kit comprenant:
- au moins deux réactifs choisis dans le groupe constitué des réactifs permettant la détermination de la présence ou l'absence d'une mutation dans le gène codant pour la Dihydropyrimidine Déshydrogénase, la mesure de la concentration en uracile plasmatique, et la mesure du rapport des concentrations plasmatiques dihydrouracile/uracile; et
- des instructions permettant la détermination de la dose de 5-fluorouracile adaptée audit patient.

Les kits de diagnostics décrits précédemment peuvent notamment comprendre comme réactifs une colonne possédant une phase stationnaire composée de particules de carbone sphériques totalement poreuses, une solution d'acétonitrile concentrée permettant d'optimiser la séparation de l'uracile et du dihydrouracile par chromatographie liquide haute performance avec détection UV, et/ou des réactifs permettant la détection de la présence ou de l'absence d'une mutation dans le gène codant pour la Dihydropyrimidine Déshydrogénase, comme par exemple des réactifs permettant l'extraction d'ADN génomique, l'amplification des fragments d'intérêt, la détermination de la taille des produits amplifiés ou leur pyroséquençage. De préférence, les kits de diagnostic contiennent entre autres au moins une des amorces pour l'amplification et/ou le pyroséquençage des fragments amplifiés ayant pour séquence nucléique SEQ ID NO : 3, 4, 9, 10, 15 à 20, 23 à 28, 31, 32, 37, 38, 40, 43, 47, 49, 50, 53 à 55, 57 ou 60. Les différents réactifs peuvent être présentés soit sous la forme d'une unité globale contenant tous les réactifs, soit sous la forme de plusieurs entités distinctes contenant des réactifs différents (en particulier si certains réactifs doivent être conservés à température ambiante et d'autres sous forme congelée).

Les instructions permettant la détermination de la dose de 5-fluorouracile adaptée audit patient peuvent se présenter sous différentes formes. Elles comprennent de préférence une liste des mutations significatives du gène codant pour la DPD ainsi que des valeurs de références ou valeurs seuils permettant la mise en oeuvre du diagnostic, et auxquelles peuvent être comparées les valeurs éventuellement mesurées de la concentration en uracile plasmatique et/ou du rapport des concentrations plasmatiques dihydrouracile/uracile. De manière avantageuse, la liste des mutations significatives du gène codant pour la DPD comprend les mutations I560S, IVS14+1G>A, D949V, L155Stop, R21Stop, Del TCAT295, Del T812, Del TG1039, E386Stop, et Del C1897; une valeur seuil pour la concentration en uracile plasmatique est 15 □g/L ; et des valeurs seuils pour le rapport des concentrations plasmatiques dihydrouracile/uracile sont 1 ; 3 ; et/ou 6.

Dans un mode de réalisation avantageux, les instructions contenues dans lesdits kits comprennent également un algorithme de décision. De préférence l'algorithme de décision est celui décrit précédemment.

La présente description concerne également une méthode de traitement d'un patient souffrant d'une maladie qui peut être traitée par du 5-fluorouracile, de préférence un cancer, ladite méthode comprenant :
(a) la collecte des résultats de la détermination de la présence ou de l'absence d'une mutation dans le gène codant pour la Dihydropyrimidine Déshydrogénase, de la mesure de la concentration en uracile plasmatique, et éventuellement de la mesure du rapport des concentrations plasmatiques dihydrouracile/uracile; et
(b) l'injection d'une dose de 5-fluorouracile adaptée au patient en fonction des résultats obtenus à l'étape (a).

Une telle méthode de traitement peut également comprendre les étapes suivantes :
(c) la mesure à partir d'au moins un prélèvement sanguin de la concentration plasmatique en 5-fluorouracile au cours des jours/heures suivants une injection de 5-fluorouracile, et
(d) l'adaptation éventuelle de la dose suivante de 5-fluorouracile injectée au patient en fonction du résultat de l'étape (c).

La description concerne encore l'utilisation d'au moins une amorce dont la séquence nucléique est l'une des SEQ ID NO : 3, 4, 9, 10, 15 à 20, 23 à 28, 31, 32, 37 et 38 pour l'amplification de fragments du gène codant pour la DPD.

La description concerne également l'utilisation d'au moins une amorce dont la séquence nucléique est l'une des SEQ ID NO : 40, 43, 47, 49, 50, 53 à 55, 57 et 60 pour le pyroséquençage de fragments du gène codant pour la DPD.

Les avantages de la présente invention sont illustrés plus clairement dans les figures 1 et 2 et les exemples ci-après.
**Figure 1****.** Algorithme de décision pour l'évaluation d'une dose de 5-FU adaptée à un patient. DS : dose standard
   (1) : sensibilité accrue : non, prédisposition au développement d'une toxicité : non
   (2) : sensibilité accrue : oui, prédisposition au développement d'une toxicité : oui
   NB : attention particulière en cas d'uracile plasmatique > 100 µg/L et/ou de mutation significative homozygote et ou de rapport UH₂/U < 1.
**Figure 2****.** Principe de la technique de pyroséquençage.
**Figure 3****.** Localisation des différentes mutations étudiées sur le gène de la DPD.
   Fe-S : sites catalytiques apportant les électrons nécessaires aux réactions d'oxydoréductions, FAD : site de liaison au flavine adénine dinucléotide (FAD),
   NAPDH : site de liaison au nicotinamide adénine dinucléotide phosphate (NADPH)

Les mutations significatives sont indiquées en gras.

### EXEMPLE 1: Patients étudiés et toxicités au 5-FU

Les différents paramètres (mutation significative, concentration plasmatique en uracile, rapport des concentrations plasmatiques dihydrouracile/uracile) ont d'abord été étudiés sur 250 patients. Dans certains cas, une étude à plus grande échelle a ensuite été réalisée.

Ces 250 patients étaient traités pour des cancers colorectaux avec des protocoles comprenant du 5-fluorouracile (LV5FU2 simplifié ou perfusion hebdomadaire de 4 heures, tel que décrit précédemment).

Les éventuelles toxicités induites par le traitement au 5-FU ont été analysées après une première ou deuxième cure, afin de dépister les patients à risques de toxicités immédiates graves au 5-FU. Tous les résultats présentés dans les exemples suivants concernent donc des toxicités développées suite à une première ou deuxième cure de 5-FU. Les toxicités de type hématologique, cutanées, les diarrhées et les vomissements ont été relevés, et leur importance a été quantifiée selon un grade de 0 à IV. La signification des différents grades de toxicité au 5-FU tels que définis par l'OMS est présentée dans le Tableau 2.

**Tableau 2. Définition OMS des différents grades de toxicité selon les régions atteintes.**

| **Type de toxicité** | **Grade 0** | | **Grade I** | | | **Grade II** | | | **Grade III** | | | **Grade IV** | |
|---|---|---|---|---|---|---|---|---|---|---|---|---|---|
| **Hématologiques** | | | | | | | | | | | | | |
| | | | | | | | | | | | | | |
| Hémoglobine | | | | | | | | | | | | | |
| | ≥ | 11 | 9,5 | - | 10,9 | 8 | - | 9,4 | 6,5 | - | 7,9 | < | 6,5 |
| g/100ml | ≥ | 4 | 3,0 | - | 3,9 | 2,0 | - | 2,9 | 1,0 | - | 1,9 | < | 1,0 |
| Leucocytes x10 | ≥ | 2 | 1,5 | - | 1,9 | 1,0 | - | 1,4 | 0,5 | - | 0,9 | < | 0,5 |
| Poly. | ≥ | 100 | 75 | - | 99 | 50 | - | 74 | 25 | - | 49 | < | 25 |
| Neutrophiles x 10 Plaquettes x 100 | Absence | | Pétéchies | | | Modérée | | | Moyenne | | | Importante | |
| Hémorragie | | | | | | | | | | | | | |
| **Cutanées** | Aucune | | Erythème | | | Desquamation sèche, vésicules, prurit | | | Desquamation humide, ulcération | | | Nécrose nécessitant une exérèse chirurgicale | |
| **Diarrhées** | Aucune | | Passagère < 2 jours | | | Tolérable > 2 jours | | | Intolérable requérant un traitement | | | Déshydratation / diarrhée hémorragique | |
| **Vomissements** | | | | | Aucun | Un épisode En 24 h | 2-5 épisodes en 24 h | 6-10 épisodes en 24 h | | | | | > 10 épisodes en 24 h ou nécessité de perfusion |

### EXEMPLE 2: Concentration en uracile plasmatique et déficit enzymatique de la DPD

### 2.1 Mesure de la concentration en uracile et dihydrouracile plasmatique

Une méthode de dosage des concentrations en uracile et en dihydrouracile plasmatique par chromatographie liquide haute performance avec détection UV (barrettes de diodes) a été développée. On utilise une colonne Hypercarb™ dont la phase stationnaire est composée de particules de carbone sphériques totalement poreuses. Sur ce type de colonne, plus l'analyte est polaire, plus son interaction avec la phase stationnaire est forte, conduisant ainsi à un temps de rétention supérieur à celui obtenu avec les autres types de colonnes. Avec une telle colonne, l'utilisation d'une phase mobile contenant de l'acétonitrile à différents pourcentages permet d'optimiser la séparation de l'uracile et du dihydrouracile des autres composés présents dans l'extrait plasmatique. La pureté des pics chromatographiques a été vérifiée à l'aide du détecteur à barrette de diodes.

La méthode développée est fiable, reproductible et linéaire sur les gammes de concentrations testées : de 6,25 à 100 □g/L pour l'uracile et de 12,5 à 200 □g/L pour le dihydrouracile. Les limites de quantification déterminées à 1,25 et 0,625 respectivement pour l'uracile et le dihydrouracile sont inférieures aux concentrations physiologiques.

### 2.2. Résultats

Une grande variabilité interindividuelle des concentrations plasmatiques d'uracile a été observée. Les deux patients ayant présenté les plus fortes toxicités (ayant conduit au décès du patient dans un cas et à 40 jours d'hospitalisation dans l'autre cas) présentaient des taux d'uracile très supérieurs aux autres patients (230 et 28 fois supérieurs à la médiane). De plus, nous avons pu mettre en évidence l'existence d'une corrélation significative entre la toxicité et la concentration d'uracile plasmatique (p<0,001). En effet, les concentrations moyennes d'uracile augmentent avec le grade de toxicité. Elles sont notamment plus élevées pour les toxicités de grades III et IV. De plus, aucun des patients ayant présenté une toxicité ne possédait un taux d'uracile inférieur à 10 □g/L De même, aucun patient présentant un taux inférieur à 15 n'a présenté de toxicité sévère (grade III ou IV). Parmi les dix sept patients ayant présenté une toxicité de grade III ou IV, treize (76,5%) possédaient un taux d'uracile supérieur à 20 □g/L, et quinze (88%) un taux supérieur à 15 □g/L. Une corrélation significative et négative a également été mise en évidence entre la concentration plasmatique d'uracile et la clairance du 5-FU (r=-0,221 ; p=0,006). Ainsi, les taux élevés d'uracile retrouvés chez les patients ayant présenté une toxicité semblent liés à un déficit du catabolisme des pyrimidines. En effet, une activité réduite de la DPD pourrait être à l'origine d'une accumulation d'uracile, celui-ci n'étant plus que faiblement métabolisé en dihydrouracile.

Cependant, tous les patients possédant une concentration élevée d'uracile n'ont pas présenté de toxicité, suggérant ainsi que cette concentration n'est pas uniquement dépendante du catabolisme des pyrimidines. Ainsi, l'approche consistant à quantifier l'uracile plasmatique ne permet pas à elle seule de déterminer les patients susceptibles de présenter une toxicité au 5-FU. Une diminution systématique de dose à la première cure pour ces patients entraînerait un sous-dosage chez plusieurs d'entre eux, induisant une diminution de l'efficacité du traitement. Cependant, le dosage de l'uracile plasmatique pourrait permettre d'effectuer une première évaluation des risques de toxicité du patient. En effet, les patients possédant un taux d'uracile faible (inférieur à 15) ont un risque très réduit de présenter une toxicité sévère. Une diminution de dose de 5-FU ne semble donc pas nécessaire dans ce cas. Pour les autres patients, une étude plus approfondie des capacités métaboliques du patient semble indispensable.

Les différents résultats sont synthétisés dans le Tableau 3.

**Tableau 3. Concentration en uracile plasmatique et développement d'une toxicité sévère après traitement avec du 5-FU.**

| **Concentration en uracile plasmatique** | **Nombre de patients** | **Nombre de patients avec une toxicité de grade III-IV** | **% de patients avec une toxicité de grade III-IV** | **% de patients sans toxicité de grade III-IV** |
|---|---|---|---|---|
| **< 15 µg/L** | 164 | 2 | 1,21 | 98,8 |
| **> 15 µg/L** | 86 | 15 | 17,44 | 82,55 |

### EXEMPLE 3: Rapport dihydrouracile/uracile plasmatique et déficit enzymatique de la DPD

### 3.1 Evaluation de la stabilité du rapport dihydrouracile/uracile plasmatique

Le prélèvement sanguin n'étant pas toujours effectué dans la même structure que l'analyse de l'échantillon, la première étape a consisté à évaluer la stabilité du rapport dihydrouracile/uracile dans différentes conditions: le délai entre le prélèvement et la centrifugation de l'échantillon sanguin, la température de conservation du plasma, et l'heure et le jour de prélèvement de l'échantillon sanguin.

Les résultats obtenus montrent que le rapport dihydrouracile/uracile:
1. n'est pas modifié quel que soit le délai de 0 à 8h entre le prélèvement et la centrifugation de l'échantillon sanguin,
2. est stable si le plasma est conservé à -20°C, mais diminue au cours du temps si le plasma est conservé à +4°C ou à température ambiante,
3. varie peu d'un jour à l'autre mais en fonction de l'heure du prélèvement.

Au vu de ces résultats, l'analyse qui suit a été réalisée sur des échantillons sanguins prélevés entre 8 et 10 heures du matin et dont le plasma a été conservé à -20°C.

### 3.2 Résultats

L'étude de la distribution du rapport dihydrouracile/uracile (UH2/U) a montré qu'il existait une grande variabilité interindividuelle des rapports, avec une moyenne de 7,6±2,7 [0,002 - 17,3].

Afin d'évaluer la pertinence du rapport UH2/U comme facteur prédictif de la toxicité au traitement, la corrélation du rapport avec la toxicité et la clairance plasmatique du 5-FU a été étudiée. Concernant la clairance plasmatique du 5-FU, aucune corrélation avec le rapport UH2/U n'a pu être mise en évidence dans la totalité de la population, quel que soit le protocole étudié. Par contre, lorsque seuls les rapports inférieurs à la médiane (7,3) sont pris en compte, une corrélation significative a été retrouvée, (r=0,301 ; p=0,016).

L'évaluation de la corrélation entre le rapport UH2/U U et la toxicité est concluante. En effet, une corrélation significative a été mise en évidence avec la toxicité (p<0,001), avec une diminution du rapport moyen lorsque le grade de toxicité augmente. De plus, parmi les 17 patients ayant présenté une toxicité sévère, de grade III ou IV, 64,7% (11 patients) possédaient un rapport UH2/U inférieur à 5 et 82,4% (14 patients) un rapport inférieur à 6. Cependant, tous les patients possédant un rapport faible n'ont pas présenté de toxicité au traitement. Ainsi, parmi les 232 patients ayant bien toléré leur traitement, 24 (10,3%) possédaient un rapport UH2/U inférieur à 5. Malgré ce résultat, le rapport UH2/U pourrait permettre de compléter les résultats obtenus avec la mesure du taux d'uracile plasmatique, les patients présentant un rapport inférieur à 6 possédant un risque accru de toxicité au 5-FU.

Les différents résultats sont synthétisés dans le Tableau 4.

**Tableau 4. Rapport dihydrouracile/uracile plasmatique et développement d'une toxicité sévère après traitement avec du 5-FU.**

| **Rapport UH2/U** | **Nombre de patients** | **Nombre de patients avec une toxicité de grade III-IV** | **% de patients avec une toxicité de grade III-IV** | **% de patients sans toxicité de grade III-IV** |
|---|---|---|---|---|
| **< 6** | 64 | 14 | 21,87 | 78,12 |
| **> 6** | 186 | 3 | 1,6 | 98,4 |

### EXEMPLE 4: Polymorphisme génétique dans le gène codant pour la DPD et déficit enzymatique de la DPD

### 4.1 Mutations connues dans le gène de la DPD

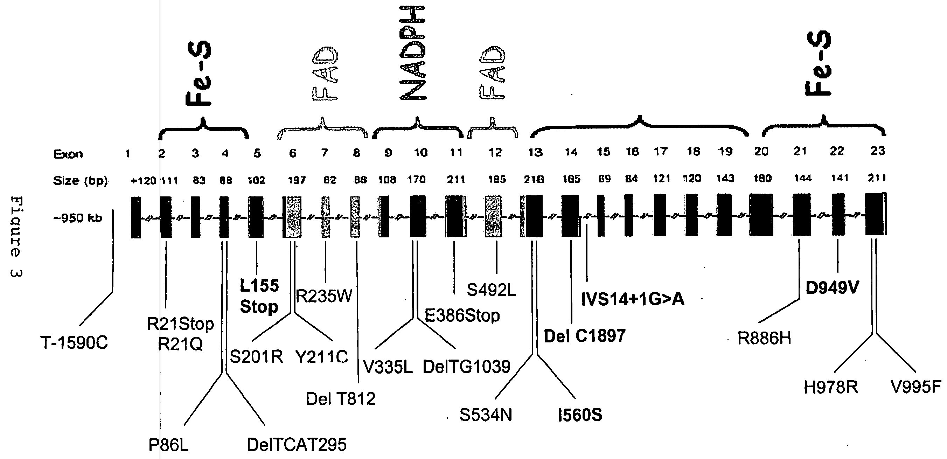

Bien que la majorité des mutations identifiées à ce jour sur le gène de la DPD résultent en un changement d'acides aminés dans la phase codante, toutes ces mutations ne sont pas spécifiquement retrouvées chez des patients présentant une activité diminuée en DPD (Ridge et al. 1998. Br. J. Clin. Pharmacol., 1998, 46(2), 151-156 ; Milano et al. 2000. European journal of cancer 36, 37-42). C'est pourquoi une méthode de génotypage a été mise au point par les inventeurs pour les 22 principales mutations susceptibles d'être liées à un déficit en DPD. La Figure 3 représente l'emplacement de ces mutations sur le gène de la DPD.

Les caractéristiques et les conséquences avérées ou possibles de ces 22 mutations sont résumées dans le Tableau 5.

**Tableau 5. Caractéristiques des mutations étudiées**

| **Mutation** | **Position dans le gène** | **Fréquence hétérozygote estimée (%)** | **Conséquence connue ou présumée** | **Publication associée** |
|---|---|---|---|---|
| **T-1590C** | promoteur | < 0,5% | Située au niveau d'un élément de réponse à l'interféron gamma, pourrait influencer l'expression du gène de la DPD | 1 |
| **R21 Stop (=C61T)** | exon 2, codon 21 | ND | Protéine tronquée avant les principaux sites de liaison aux co-enzymes et au substrat => activité nulle en DPD ? | 2 |
| **R21Q (=G62A)** | exon 2, codon 21 | ND | A priori, pas de conséquence sur l'activité enzymatique de la DPD | 2 |
| **C29R (=T85C)** | exon 2, codon 29 | 29 à 33% | A priori, pas de conséquence sur l'activité enzymatique de la DPD | 3 |
| **P86L (=C257T)** | exon 4, codon 86 | ND | Changement conformationnel complet => un déficit complet en DPD. | 4 |
| **Del TCAT295** | exon 4, nucléotides 295-298 | ND | Codon stop prématuré => déficit complet ? | 5 |
| **S201R (=A601C)** | exon 6, codon 201 | ND | Perturbation du site enzymatique => déficit complet en DPD. | 4 |
| **Y211C (=A632G)** | exon 6, codon 211 | ND | Changement conformationnel déstabilisant l'enzyme => déficit partiel? | 4 |
| **R235W (=C703T)** | exon 7, codon 235 | ND | Protéine mutante a une activité résiduelle de 1% chez E. Coli | 3 |
| **Del T812** | exon 8, nucléotide 812 | ND | codon stop => une protéine tronquée inactive | 6 |
| **V335L (=G1003T)** | exon 10, codon 335 | 25 | Modification du site de liaison au NADPH. Activité résiduelle chez E. coli de 15% | 7 |
| **Del TG1039** | exon 10, nucléotides 1039-1042 | ND | codon stop prématuré => activité nulle | 8 |
| **E386Stop (=G1156T)** | exon 11, codon 386 | ND | codon stop prématuré => activité nulle | 1 |
| **S492L (=C1475T)** | exon 12, codon 492 | ND | changement conformationnel de la protéine => déficit complet en DPD ? | 4 |
| **S534N (=G1601A)** | exon 13, codon 534 | 0,8 | activité enzymatique réduite ? | 1 |
| **I560S (=T1679G)** | exon 13, codon 560 | ND | changement conformationnel => déficit partiel ou complet | 1 |
| **Del C1897** | exon 14, nucléotide 1897 | ND | codon stop au niveau du site de liaison au substrat => déficit complet chez un patient | 3 |
| **IVS14+1G>A** | intron 14 | 1,5 | délétion complète de l'exon 14 lors de la transcription de l'ARN pré-messager (perte de 165 pb) => déficit complet | 9 |
| **R886H (=G2657A)** | exon 21, codon 886 | ND | activité enzymatique résiduelle de 25% che E. coli. | 3 |
| **D949V (=A2846T)** | exon 22, codon 949 | ND | diminution très probable de l'activité de la DPD | 4 |
| **H978R (=A2933G)** | exon 23, codon 978 | ND | déficit complet ? | 4 |
| **V995F (=G2983T)** | exon 23, codon 995 | ND | changement conformationnel de la DPD=> activité enzymatique nulle ? | 10 |

### Références :

1. Collie-Duguid et al., 2000. Pharmacogenetics 10 , 217-223.
2. Van Kuilenburg et al., 2003. Ann Oncol 14, 341-342
3. Vrecken et al.,1997. Hum. Genet. 101, 333-338
4. Van Kuilenburg et al., 2002. Biochem. J. 364, 157-163.
5. Vrecken et al.,1997. Hum. Genet. 100, 263-265.
6. Yamaguchi et al. en 2001. Jpn. J. Cancer Res. 92, 337-342.
7. Dobritzsch et al., 2001. The EMBO journal 20, 650-660
8. Van Kuilenburg et al., 2000. Adv Exp Med Biol. 486:247-50.
9. Wei et al., 1996. The journal of clinical investigation 98, 610-615.
10. Van Kuilenburg et al., 1999. Hum Genet.104(1):1-9.

### 4.2 Recherche de nouvelles mutations

La liste des mutations décrites dans la littérature n'est pas exhaustive. D'autres mutations encore inconnues sur le gène de la DPD et susceptibles d'engendrer un déficit enzymatique peuvent être présentes. C'est pourquoi d'autres mutations éventuelles ont été recherchées chez des patients ne présentant pas les mutations testées préalablement et ayant néanmoins présenté un épisode de toxicité aiguë au 5-FU. Deux stratégies de recherche ont été utilisées :
- l'amplification puis séquençage classique des 23 exons du gène de la DPD.
- L'utilisation d'un test fonctionnel dit "test de la protéine tronquée".

Le test de la protéine tronquée a pour but de rechercher des mutations engendrant un codon stop, donc une protéine anormalement tronquée. Son principe consiste principalement en la synthèse de la protéine. En effet, lorsqu'une partie importante de la séquence codante peut être amplifiée par PCR sur l'ARN messager, ou sur un exon de grande taille, un premier criblage peut être effectué par transcription et traduction *in vitro* de la protéine. Cette technique se déroule en 3 grandes étapes :
- l'extraction des ARNs messagers codant pour la protéine à synthétiser,
- la synthèse et amplification de l'ADN complémentaire, et
- la synthèse de la protéine.

Afin de synthétiser la protéine, l'ADNc total ou une partie de ce dernier est cloné dans un vecteur d'expression. Les protéines ainsi synthétisées sont ensuite déposées dans un gel SDS-PAGE. La révélation du gel peut se faire par western blot ou par coloration au bleu de Coomassie.

Le test de la protéine tronquée a permis de mettre en évidence une mutation susceptible d'être corrélée avec une altération de l'activité enzymatique de la DPD et le développement de toxicités graves après traitement au 5-FU : la mutation L155Stop (=T464A), puisque cette mutation provoque une protéine tronquée

### 4.3 Analyse de la présence ou l'absence de ces mutations

### 4.3.1 Isolement des leucocytes

L'analyse a été réalisée à partir de prélèvements sanguins. A 500 µL de sang total est ajouté 1 ml de solution de lyse (Red blood cell lysis buffer, ROCHE, Mannheim, Allemagne). Cette solution permet la lyse sélective des globules rouges. Le tube est homogénéisé par retournement pendant 10 minutes à 45 rpm (tours par minute), puis centrifugé 5 minutes à 1300 g à température ambiante. Un culot blanc de leucocytes est alors observé au fond du tube. Après élimination du surnageant, ce culot est repris avec 500 µL de solution de conservation des acides nucléiques (RNA/DNA stabilization reagent for blood/bone marrow, ROCHE, Mannheim, Allemagne). Cette dernière solution permet également la lyse des globules blancs. Ce tube numéroté et identifié, est ensuite conservé à - 20° C.

### 4.3.2 Extraction de l'ADN génomique

Cette étape d'extraction est réalisée à l'aide du kit *"DNA isolation Kit for blood*/*bone marrow*/*tissue* " (Roche, Allemagne).

### 4.3.3 Préparation de l'ADN complémentaire

L'extraction des ARNs messagers totaux est réalisée à l'aide du kit « High Pure RNA Isolation Kit » (Roche, Allemagne). L'ADN complémentaire (ADNc) est ensuite synthétisé par transcription inverse par le protocole suivant : à 12 µL d'ARNs totaux sont ajoutés 1 µL d'amorces aléatoires (2,65 µg/µL). Ce mélange est ensuite incubé à 70° C pendant 5 minutes afin d'éliminer les structures secondaires des ARNs, et plongé immédiatement après à 4° C afin d'empêcher la renaturation des brins d'ARNs. Puis un milieu réactionnel composé de tampon 1 X (50 mM Tris-HCl, 75 mM KCl, 3 mM MgCl₂, 10 mM DTT ; Promega, Madison, USA), 0,25 mM de chaque dNTP, 1µL d'inhibiteur des ribonucléases (RNasin 40 µg/µL ; Promega, Madison, USA) et de la rétrotranscriptase M-MLV RT (Moloney Murine Leukemia Virus Reverse Transcriptase, 200 U/µL ; Promega, Madison, USA). Le tout est incubé pendant 1 heure à 37° C.

### 4.3.4 Amplification par PCR

L'amplification des fragments d'intérêt a été réalisée selon un protocole standard de PCR. Le Tableau 6 récapitule les amorces utilisées pour les différents fragments, ainsi que la taille attendue des produits amplifiés.

**Tableau 6. Amorces utilisées pour l'amplification des différents fragments d'intérêt**

| **Mutation** | **Amorce sens** | **Amorce anti-sens *** | **Taille du produit amplifié** |
|---|---|---|---|
| T-1590C | atccaggactctccccagat (SEQ ID NO : 1) | atccaggactctccccagat (SEQ ID NO : 2) | 203 |
| R21Stop | gtctttagagtatcctggcttt (SEQ ID NO : 3) | tcttatcaggatttcttttccaatg (SEQ ID NO : 4) | 110 |
| R21Q | gtctttagagtatcctggcttt (SEQ ID NO : 3) | tcttatcaggatttcttttccaatg (SEQ ID NO : 4) | 110 |
| C29R | cctggctttaaatctcgaa (SEQ ID NO : 5) | tcttatcaggatttcttttccaatg (SEQ ID NO: 6) | 100 |
| P86L | gagatgcctgaaatgtgc (SEQ ID NO : 7) | ttgtttgcaatacttgtgatg (SEQ ID NO : 8) | 150 |
| Del TCAT295 | tgtcagaagagctgtccaa (SEQ ID NO : 9) | ttgtttgcaatacttgtgatg (SEQ ID NO : 10) | 62 |
| S201 R | ccacagatcagaaatccttc (SEQ ID NO : 11) | accaccaacatattcttgttt (SEQ ID NO : 12) | 175 |
| Y211C | ccacagatcagaaatccttc (SEQ ID NO : 11) | accaccaacatattcttgttt (SEQ ID NO : 12) | 175 |
| R235W | acttctgaaattcctcagttc (SEQ ID NO : 13) | ctttacaccaaggtccttca (SEQ ID NO : 14) | 77 |
| Del T812 | agataatttgcggtaaaagc (SEQ ID NO : 15) | agcagctttgtagccttttt (SEQ ID NO : 16) | 90 |
| V335L | agataatttgcggtaaaagc (SEQ ID NO : 15) | agcagctttgtagccttttt (SEQ ID NO : 16) | 90 |
| Del TG1039 | cctgtcactctccattgc (SEQ ID NO : 17) | tcctcagggacagctcttat (SEQ ID NO : 18) | 200 |
| E386Stop | tggaacttgctaaggaagaaaa (SEQ ID NO : 19) | aaccaaaggcactgatgacc (SEQ ID NO : 20) | 204 |
| S492L | gtatttgcaggtggtgatgt (SEQ ID NO : 21) | ctgtacgtatttgtgaatgtacc (SEQ ID NO : 22) | 101 |
| S534N | aatatggagcttccgtttct (SEQ ID NO : 23) | gagagaaagttttggtgagg (SEQ ID NO : 24) | 204 |
| I560S | aatatggagcttccgtttct (SEQ ID NO : 23) | gagagaaagttttggtgagg (SEQ ID NO : 24) | 204 |
| Del C1897 | ctttcatcaggacattgtga (SEQ ID NO : 25) | caccaacttatgccaattct (SEQ ID NO : 26) | 222 |
| IVS14+1G>A | atcagtgagaaaacggctgc (SEQ ID NO : 27) | taaacattcaccaacttatgcca (SEQ ID NO : 28) | 150 |
| R886H | tctgacctaacatgcttc (SEQ ID NO : 29) | aggccttttggggataaaac (SEQ ID NO : 30) | 200 |
| D949V | aagcactgcagtaccttggaa (SEQ ID NO : 31) | tcatgtagcatttaccacagttga (SEQ ID NO : 32) | 107 |
| H978R | ccctctatttctgtttgcag (SEQ ID NO : 33) | agggtacgcctctctttggt (SEQ ID NO : 34) | 175 |
| V995F | ccctctatttctgtttgcag (SEQ ID NO : 35) | agggtacgcctctctttggt (SEQ ID NO : 36) | 175 |
| L155Stop | Ttatggagctgctaagatga (SEQ ID NO : 37) | atcatacatacctcagtagcaaa (SEQ ID NO : 38) | 200 |

| | | | |
|---|---|---|---|
| * Dans le cas où le produit amplifié est ensuite analysé par pyroséquençage, alors l'amorce anti-sens utilisée pour l'amplification est biotinylée en 5'. | | | |

### 4.3.5 Détermination de la taille des produits amplifiés

Tous les produits d'amplification (8 µl) sont séparés sur gel d'agarose 2% (Invitrogen, USA) par migration électrophorétique en présence de bromure d'éthidium (0,5µg/mL) (Sigma-Aldrich^{®}, USA). L'amplification et la taille des fragments sont alors visualisées sous U.V. et comparées à un marqueur de poids moléculaire (Step Ladder 50pb ou Lamba DNA BSTEII, Sigma-Aldrich^{®}, USA).

### 4.3.6 Pyroséquençage des produits amplifiés

Le principe de la technique de pyroséquençage est décrit sur la Figure 2.

Après l'amplification des fragments d'intérêt avec les amorces antisens biotinylées en 5', les produits sont préparés pour la réaction de pyroséquençage proprement dite. La préparation se fait grâce à un système de filtration par le vide développée par Biotage AB. 20 µL de produit de PCR sont immobilisés sur des billes de sépharose recouvertes de streptavidine (*Streptavidin Sepharose*™ *High Purity*, Amersham Biosciences, Suède). En présence un tampon de fixation (10mM Tris Base,2M NaCl, 1mM EDTA, 0,1% Tween20) à pH 7,6, l'affinité avidine/biotine permet la fixation et l'immobilisation du produit biotinylé sur les billes. Ce complexe insoluble peut alors être aspiré sur des filtres par le système de vide permettant un lavage des échantillons. Cet ADN est ensuite dénaturé par une solution de soude 0,2M puis lavé dans une solution de Tris 10mM, pH 7,6. Seul le brin contenant la biotine reste fixé sur les billes. Le complexe ADN simple brin/sépharose est alors remis en suspension dans 50 µL de tampon d'hybridation auquel sont ajoutées 15 pmol d'amorce de séquence. 2 min à 80°C sont nécessaires afin d'éliminer les éventuelles structures secondaires de l'ADN et permettre une meilleure fixation de l'amorce.

La réaction de séquençage est effectuée à 28°C dans un appareil de type PSQ 96MA™ (Biotage AB, Suède). Les enzymes sont ajoutées à l'ADN, puis les substrats et ensuite les nucléotides un à un de manière automatique par une cartouche de distribution. Les volumes sont déterminés selon le nombre d'échantillons à analyser. Il faut compter par échantillon : 5 µL d'enzymes et 5 µL de substrats + 50 µL de volume mort. Pour les nucléotides, 0,2 µL de chaque nucléotide par base à tester sont ajoutés aux 50 µL de volume mort.

L'ordre d'ajout des nucléotides est déterminé selon la position de l'amorce, la séquence du brin complémentaire et la mutation à détecter. Cette séquence ne dépasse pas en règle générale 10 à 15 nucléotides. Dans tous les cas, la séquence d'ordre d'ajout des nucléotides commence par un nucléotide contrôle n'ayant pas son complémentaire sur le fragment pyroséquencé. Ce dernier sert de témoin négatif, aucun pic ne doit apparaître. Ensuite, les nucléotides correspondant à la séquence complémentaire au fragment amplifié sont ajoutés un à un. Au niveau de la mutation, les deux nucléotides correspondant aux deux génotypes possibles sont ajoutés successivement. Le pyrogramme™ obtenu permet alors de déterminer quel est le génotype de l'individu (homozygote 1, hétérozygote ou homozygote 2). Si une deuxième mutation doit être détectée, un autre nucléotide contrôle n'ayant pas son complémentaire sur le fragment pyroséquencé est ajouté avant de passer à la suite de la séquence.

Par exemple, pour le pyroséquençage de la mutation IVS14+1G>A, la séquence analysée est CAAC(G/A)TAA. L'ordre de distribution des dNTPs pour cette mutation est alors G, C, A, C, G, A, C, T, A. En effet, le premier nucléotide G est un nucléotide contrôle, les cinq nucléotides suivants (C, A, C, G, A) permettent de détecter ou non la présence de la substitution G>A, le nucléotide C suivant est un nucléotide contrôle, et les deux derniers nucléotides T et A permettent de séquencer les trois nucléotides suivants de la séquence à analyser (voir Tableau 6).

Le choix de l'amorce de séquence est très important pour la détection des mutations par la méthode de pyroséquençage. Pour une meilleure séquence, cette amorce ne doit pas être placée à plus de 5 bases de la mutation et le complexe entre le brin d'ADN à séquencer et l'amorce de séquence doit posséder une stabilité thermodynamique suffisante. La température de la réaction ne pouvant être changée, des oligonucléotides modifiés ont été utilisés pour le pyroséquençage de certains fragments. Ces oligonucléotides modifiés comprennent au moins un nucléotide contenant un pont méthylène 2'O-4'C qui permet de maintenir ledit nucléotide dans une conformation bicyclique rigide. Les amorces de séquence finalement retenues et utilisées sont présentées dans le Tableau 7.

**Tableau 7. Récapitulatif des amorces utilisées pour le pyroséquençage.**

| **Mutation** | **Amorce de pyroséquençage** | **Séquence à analyser^{a}** | **Ordre d'injection des dNTPs^{b}** |
|---|---|---|---|
| T-1590C | atttgcaaaggtaagatact (SEQ ID NO : 39) | t(T/C)aaccac | ctcgacac |
| R21Stop | cctggctttaaatcct (SEQ ID NO : 40) | (C/T)gaacac | GCTCGATCAC |
| R21Q | cctggctttaaatcct (SEQ ID NO : 40) | C(G/A)AACAC | GCTCGATCAC |
| C29R | caaactcatgcaactctg (SEQ ID NO : 41) | (T/C)gttcc | gtcagtc |
| P86L | aatgtgcagatgccc (SEQ ID NO : 42) | (C/T)GTGTC | GCTCGTGTC |
| Del TCAT295 | aactaatcttgatattaa (SEQ ID NO : 43) | A[TCAT]TCATCACAA | GATCATCATCA |
| S201R | tgggcctgcaagtata (SEQ ID NO : 44) | (A/C)GTTGTG | TACTGTGTG |
| Y211C | ttggctcgattgggg (SEQ ID NO : 45) | T(A/G)CTCT | GTAGTCTCT |
| R235W | gaaattcctcagttc (SEQ ID NO : 46) | (C/T)GGCTGCC | GCTCGCTGC |
| Del T812 | aatgaaatgactcttagcac (SEQ ID NO : 47) | TT[T]GAAAG | CTCGAG |
| V335L | tcgatacggggagtc (SEQ ID NO : 48) | (G/T)TGATCG | CGTCGATCG |
| Del TG1039 | Gagacactgcctttgac (SEQ ID NO : 49) | [TG]TGCAAC | GTGTGTCAC |
| E386Stop | tgctaaggaagaaaagtgt (SEQ ID NO : 50) | (G/T)aatttc | cgtcatc |
| S492L | ggctaacactacagtggaat (SEQ ID NO : 51) | (C/T)GGTG | GCTCGTG |
| S534N | tattgatctggtggacatta (SEQ ID NO : 52) | (G/A)TGTA | CGACTGTA |
| I560S | ccagccaccagcacatcaa (SEQ ID NO : 53) | TGA(T/G)TCG | CTGAGTGCG |
| Del C1897 | CCAACTAATCTTGATATTAA (SEQ ID NO : 54) | CTTT[C]CAG | GCTCTAG |
| IVS14+1G>A | aggctgactttccaga (SEQ ID NO : 55) | caac(G/A)taa | gcacgacta |
| R886H | ttggaccttatctggaa (SEQ ID NO : 56) | cagc(G/A)caag | acagcgatcag |
| D949V | gcaagttgtggctatga (SEQ ID NO : 57) | ttg(A/T)tgaag | atgatcgag |
| H978R | acagtttgatccagaaaccc (SEQ ID NO : 58) | (A/G)CCTGCCC | TAGACTGC |
| V995F | gctgtactctgtgtctcagt (SEQ ID NO : 59) | (G/T)tttgccc | cgtcgct |
| L155Stop | cccattaatattggt (SEQ ID NO : 60) | GGAT(T/A)GCA | CGATATGCA |

| | | | |
|---|---|---|---|
| ^{a} Les substitutions sont indiquées en gras entre parenthèses, le nucléotide sauvage en premier, le nucléotide muté en second. Pour les délétions, la partie absente en cas de délétion est indiquée en gras entre crochets ^{b} Les nucléotides contrôles, n'ayant pas de base complémentaire sur le fragment pyroséquencé, sont indiqués en gras | | | |

### 4.3.7 Réalisation de témoins négatifs et positifs

Afin de valider la réaction de mini-séquençage luminométrique, des témoins ont été construits pour les mutations les plus recherchées. Pour les mutations pour lesquelles un ou plusieurs patients hétérozygotes ont été détectés, les témoins positifs et négatifs ont été obtenus par clonage dans pGEM-T. Les clones ont ensuite été séquencés par mini-séquençage luminométrique afin de discriminer les homozygotes pour la mutation et les sauvages. En effet, lors du clonage chacun des 2 allèles est incorporé dans un clone différent. De cette manière, les témoins sauvages et homozygotes mutés sont obtenus. Le témoin hétérozygote muté est construit en mélangeant en quantité équimolaire les plasmides sauvages et homozygotes mutés. Les témoins des mutations pour lesquelles aucun individu n'a été retrouvé muté ont été obtenus par mutagénèse dirigée.

### 4.4 Corrélation entre la présence de ces mutations et le développement de toxicités graves après traitement au 5-FU.

Seules les toxicités apparues après les 1^{ers} et 2^{èmes} cycles de chimiothérapies ont été prises en compte afin de dépister les patients à risques de toxicités immédiates graves au 5-FU.

### 4.4.1 Etude de 10 mutations principales

Dans un premier temps, l'étude a porté essentiellement sur 9 mutations décrites dans la littérature et sur la nouvelle mutation L155Stop mise en évidence par les inventeurs.

La présence ou l'absence de ces mutations et leur corrélation éventuelle avec une altération de l'activité enzymatique de la DPD et le développement de toxicités graves après traitement au 5-FU a d'abord été analysée sur 250 patients.

Les résultats en termes de nombre de patients, de fréquences et de retentissements cliniques et plus particulièrement toxiques, sont représentés dans le Tableau 8. La gradation selon la classification OMS est utilisée, le grade IV mettant en jeu le pronostic vital du patient.

**Tableau 8. Récapitulatif des résultats obtenus après génotypage et étude clinique des 9 mutations recherchées en priorité.**

| **Mutation** | | **Nombre de patients** | | **% de patients** | **Nombre de patients avec une toxicité de grade III-IV** | **% de patients avec une toxicité de grade III-IV** | **% de patients sans une toxicité de grade III-IV** |
|---|---|---|---|---|---|---|---|
| | Hs^{a} | | 247 | 98,8 | 15 | 6 | 93,9 |
| IVS14+1G>A | h^{b} | 250 | 3 | 1,2 | 2 | 67 | 33 |
| | Hm^{c} | | 0 | 0 | ND | ND | ND |
| | Hs | | 242 | 96,8 | 12 | 4,96 | 95 |
| D949V | h | 250 | 8 | 3,2 | 5 | 62,5 | 37,5 |
| | H | | 0 | 0 | ND | ND | ND |
| | Hs | | 247 | 98,8 | 17 | 6,88 | 93,2 |
| T-1590C | h | 250 | 3 | 1,2 | 0 | 0 | 100 |
| | H | | 0 | 0 | ND | ND | ND |
| | Hs | | 249 | 99,6 | 16 | 6,4 | 93,6 |
| I560S | h | 250 | 1 | 0,4 | 1 | 100 | 0 |
| | H | | 0 | 0 | ND | ND | ND |
| L155Stop (nouvelle mutation) | Hs | | 249 | 99,6 | 16 | 6,4 | 93,6 |
| | h | 250 | 1 | 0,4 | 1 | 100 | 0 |
| | H | | 0 | 0 | ND | ND | ND |
| Non Mutés | Hs | 250 | 238 | 95,2 | 9 | 3,8 | 96,2 |
| DelTCAT295, E386Stop, R886H, V995F | Hs | | 250 | 100 | ? | ? | ? |
| | h | 250 H 0 | 0 | 0 | ND | ND | ND |
| | H | | 0 | 0 | ND | ND | ND |

| | | | | | | | |
|---|---|---|---|---|---|---|---|
| ^{a}Hs : homozygote sauvage ^{b}h : hétérozygote ^{c}Hm : homozygote muté | | | | | | | |

Ces résultats montrent que la présence des mutations IVS14+1G>A, D949V, L155Stop et I560S est corrélée avec le développement d'une toxicité sévère après une première ou deuxième cure de 5-FU. En revanche, les 6 autres mutations testées en priorité ne semblent pas avoir d'impact sur le développement d'une toxicité sévère après une première ou deuxième cure de 5-FU.

Ces résultats ont été confirmés lors d'études portant sur un plus grand nombre de patients (voir Tableau 9)

**Tableau 9. Résultats obtenus après génotypage et étude clinique des 4 mutations impliquées dans une toxicité.**

| Mutation | | Nbre de patients | | Fréquence | % Tox III-IV |
|---|---|---|---|---|---|
| IVS141 G>A | h | 1678 | 19 | 1,13 | 62,5 |
| | Hm | | 1 | 0,06 | 100 |
| D949V | h | 1678 | 27 | 1,6 | 60 |
| | Hm | | 0 | | |
| I560S | h | 761 | 3 | 0,4 | 100 |
| | Hm | | 0 | | |
| L155Stop | h | 944 | 1 | 0,1 | 100 |
| | Hm | | 0 | | |
| Pas de SNP | Hs | 1678 | 1627 | 96,96 | ND |

| | | | | | |
|---|---|---|---|---|---|
| Hs : homozygote sauvage h : hétérozygote Hm : homozygote muté | | | | | |

Bien que la mutation L155Stop n'ait été détectée que chez un patient sur les 944 testés, le patient en question a été hospitalisé 1 semaine après une première cure de 5-FU pour cause de mucite grade IV, aplasie grade IV, syndrome mains-pieds de grade III, et de diarrhées profuses entraînant une déshydratation sévère. Son état s'est aggravé vers une insuffisance rénale et a abouti à un coma, puis au décès de ce patient. De plus, le test de la protéine tronquée avait permis de mettre en évidence que cette mutation provoque une protéine tronquée, suggérant que bien que de faible fréquence, cette mutation est corrélée avec une altération de l'activité enzymatique de la DPD et le développement de toxicités graves après traitement au 5-FU.

### 4.4.2 Etude des 13 autres mutations chez les patients non mutés pour les mutations précédentes.

Seuls les patients non mutés pour les mutations précédentes et ayant présenté un épisode de toxicité aiguë au 5-FU ont été génotypés pour les 13 autres mutations. Une trentaine de patients a donc été génotypée pour les mutations P86L, S201R, R235W, S492L, S534N, H978R, Del C 1897, F632F, R21Stop, R21Q, Y211C, delT812, délétion TG 1039-1041, et V335L.

Le Tableau 10 présente les résultats en terme de nombre de patients, de fréquences et de retentissements cliniques et plus particulièrement toxiques.

**Tableau 10. Récapitulatifs des résultats obtenus après génotypage et étude clinique pour les 13 mutations supplémentaires analysées.**

| **Mutation** | | **Nombre de patients** | | **% de patients** | **Nombre de patients avec une toxicité de grade III-IV** | **% de patients avec une toxicité de grade III-IV** | **% de patients sans une toxicité de grade III-IV** |
|---|---|---|---|---|---|---|---|
| P86L, S201R, R235W, S492L, S534N, H978R , Del | Hs^{a} | | 33 | 100,0 | 33 | 100 | 0 |
| | h^{b} | | 0 | 0,0 | 0,0 | 0,0 | 0,0 |
| | | | | | | | |
| C1897, R21Stop, R21Q, | | 33 | | | | | |
| Y211C, DelT812, Del TG1039, V335L | Hm^{c} | | 0 | 0,0 | 0,0 | 0,0 | 0,0 |

| | | | | | | | |
|---|---|---|---|---|---|---|---|
| ^{a} Hs : homozygote sauvage ^{b} h : hétérozygote ^{c} Hm : homozygote muté | | | | | | | |

D'après ces résultats, aucun patient n'a été retrouvé muté pour au moins une de ces mutations.

### 4.5 Conclusion concernant les mutations significatives

Ces différents résultats montrent que les 4 mutations IVS14+1G>A, D949V, I560S, et L155Stop sont corrélées avec une altération de l'activité enzymatique de la DPD et le développement de toxicités graves après traitement au 5-FU. Par conséquent, le dépistage de la présence d'une de ces mutations indique un risque élevé de développement d'une toxicité suite à une première ou deuxième cure de 5-FU (voir Tableau 10).

### EXEMPLE 5: Combinaisons des différents paramètres

### 5.1 Comparaison de la sensibilité et de la spécificité des différents paramètres et de leurs combinaisons.

La combinaison de plusieurs des trois paramètres a également été analysée pour le diagnostic d'une sensibilité accrue au 5-FU et donc d'une prédisposition au développement de toxicités graves après traitement au 5-FU.

Les caractéristiques en termes de sensibilité et de spécificité de ces trois paramètres, et de différentes combinaisons de ces paramètres, sont synthétisées dans le Tableau 11.

**Tableau 11. Sensibilité et spécificité des différents paramètres.**

| **Paramètre** | **Sensibilité (% faux négatifs)** | **Spécificité (% faux positifs)** |
|---|---|---|
| [U] | 5,5 | 76 |
| Rapport UH2/U | 6,6 | 69 |
| Mutation significative | 8 | 10 |
| [U] + mutation significative | 1,27 | 10 |
| Rapport UH2/U + mutation significative | 1,08 | 18,2 |
| [U] + Rapport UH2/U + mutation significative | 0,7 | 10 |

Ces résultats montrent que la combinaison d'au moins deux des paramètres étudiés précédemment de manière isolée permet un diagnostic avec une meilleure sensibilité et une meilleure spécificité que chacun de ces paramètres pris individuellement.

### 5.2 Mise en place d'un algorithme de décision

Les résultats précédents ont conduit à la mise en place d'un algorithme de décision pour le diagnostic in vitro d'une sensibilité accrue au 5-fluorouracile, de la prédisposition d'un sujet au développement d'une toxicité à la suite d'un traitement comprenant du 5-fluorouracile, et/ou la détermination de la dose de 5-FU adaptée à un patient.

Cet algorithme prend en compte les résultats déterminants des trois paramètres, à savoir :
- pour la concentration en uracile plasmatique : pour une concentration inférieure à 15 ng/mL, le risque de développer une toxicité sévère est très réduit (< 5,5%).
- pour le rapport dihydrouracile/uracile plasmatique : un rapport inférieur à 6 est fortement corrélé avec un risque accru de développer une toxicité sévère.
- pour la détection de la présence ou de l'absence d'une mutation significative dans le gène codant pour la DPD : la présence d'une mutation significative entraîne un risque fort de développer une toxicité sévère (60 à 100% en fonction de la mutation ou du génotype hétéro ou homozygote).

A partir de ces résultats, l'algorithme de décision suivant a été mis en place (voir Figure 1) :
(a) En cas
   - d'absence de mutation dans le gène codant pour la Dihydropyrimidine Déshydrogénase parmi celles recherchées et si la concentration en uracile plasmatique est inférieure à 15 µg/L (cette situation correspond à 65.2 % des patients), ou
   - d'absence de mutation dans le gène codant pour la Dihydropyrimidine Déshydrogénase parmi celles recherchées et si la concentration en uracile plasmatique est supérieure à 15 µg/l mais que le rapport UH₂/U est supérieure ou égal à 6 (cette situation correspond à 13.6 % des patients),
le sujet ne possède pas de sensibilité accrue au 5-fluorouracile, n'est pas prédisposé au développement d'une toxicité à la suite d'un traitement comprenant du 5-fluorouracile, et le traitement peut avoir lieu aux doses standard de 5-fluorouracile
(b) Dans tous les autres cas, c'est-à-dire, en cas :
   - d'absence de mutation dans le gène codant pour la Dihydropyrimidine Déshydrogénase parmi celles recherchées, si la concentration en uracile plasmatique est supérieure ou égale à 15 µg/L, et UH₂/U inférieur à 6,
   - de présence de mutation significative hétérozygote ou homozygote dans le gène codant pour la Dihydropyrimidine Déshydrogénase, sans augmentation de la concentration en uracile plasmatique,
   - de présence de mutation significative hétérozygote ou homozygote dans le gène codant pour la Dihydropyrimidine Déshydrogénase, et d'augmentation de la concentration en uracile plasmatique,
   le sujet possède une sensibilité accrue au 5-fluorouracile, est prédisposé au développement d'une toxicité à la suite d'un traitement comprenant du 5-fluorouracile, et le rapport des concentrations plasmatiques dihydrouracile/uracile est calculé pour confirmer le déficit et déterminer la dose de 5-FU à administrer. Si celui-ci est:
   (i) supérieur à 6 : la dose initiale de 5-fluorouracile est diminuée de 30% par rapport à la dose standard.
   (ii) compris entre 3 et 6 : la dose initiale de 5-fluorouracile est réduite de 50% par rapport à la dose standard et une mesure de la concentration plasmatique en 5-fluorouracile au cours des jours/heures suivant l'injection d'une dose de 5-fluorouracile est réalisée.
   (iii) compris entre 1 et 3 : la dose initiale de 5-fluorouracile est réduite de 70% par rapport à la dose standard et une mesure de la concentration plasmatique en 5-fluorouracile au cours des jours/heures suivant l'injection d'une dose de 5-fluorouracile est réalisée
   (iv) inférieur à 1 : le traitement par le 5-fluorouracile est déconseillé, même à doses réduites. Si le traitement doit malgré tout être mis en oeuvre, la dose initiale est réduite de 80% et une mesure de la concentration plasmatique en 5-fluorouracile au cours des jours/heures suivant l'injection d'une dose de 5-fluorouracile est indispensable.

De plus, en cas de mutation significative homozygote du gène codant pour la Dihydropyrimidine Déshydrogénase, d'une concentration en uracile plasmatique supérieure à 100 µg/L, et/ou d'un rapport dihydrouracile/uracile inférieur à 1, des précautions particulières de surveillance sont mises en place, même à doses réduites.

Appliqué aux patients analysés rétrospectivement, cet algorithme aurait pu permettre de décider une diminution de la dose de 5-FU pour 98,8% des patients ayant subi une toxicité sévère (grade supérieur ou égal à III) au cours des deux premières cures.

Pour les patients n'ayant pas présenté de toxicité sévère au cours des deux premières cures, l'application de l'algorithme n'aurait conduit à l'administration d'une dose réduite de 5-FU lors de la première cure que chez 8% d'entre eux. En outre, à la suite de l'adaptation clinique de dose, ou au mieux de la surveillance pharmacocinétique mise en place pour tous les patients considérés comme à risque, la dose de 5-FU aurait pu être augmentée pour la deuxième cure, ce qui aurait évité un traitement inefficace de ces patients.

### 5.4 Conclusion

Les résultats montrent que la combinaison d'au moins deux des paramètres étudiés permet de façon surprenante d'obtenir une sensibilité et une spécificité de diagnostic meilleures que la sensibilité ou la spécificité la meilleure des trois paramètres pris isolément.

La mise en oeuvre des méthodes ci-dessus pourrait donc permettre d'éviter en grande partie la survenue de toxicités sévères lors des premières cures de 5-FU administrées à des patients, notamment par la détermination d'une dose adaptée de 5-FU si une sensibilité accrue au 5-FU a été diagnostiquée.

## Revendications

1. Méthode de diagnostic in vitro de la prédisposition d'un patient au développement d'une toxicité à la suite d'un traitement comprenant du 5-fluorouracile, ladite méthode comprenant la réalisation sur un ou plusieurs échantillons biologiques dudit patient, dont au moins un prélèvement sanguin, de la combinaison : :
• de la détermination de la présence ou de l'absence d'une mutation choisie parmi IVS14+1G>A, D949V, L155Stop et I560S dans le gène de la Dihydropyrimidine Déshydrogénase, et
• de la mesure de la concentration en uracile plasmatique et/ou de la mesure du rapport des concentrations plasmatiques dihydrouracile/uracile,
le patient présentant un risque accru de développer une toxicité à la suite d'un traitement comprenant du 5-fluorouracile si :
• une mutation choisie parmi IVS14+1G>A, D949V, L155Stop et I560S est présente dans le gène de la Dihydropyrimidine Déshydrogénase et la concentration en uracile plasmatique est supérieure à 15 µg/L ; ou
• une mutation choisie parmi IVS14+1G>A, D949V, L155Stop et I560S est présente dans le gène de la Dihydropyrimidine Déshydrogénase et le rapport des concentrations plasmatiques dihydrouracile/uracile est inférieur à 6 ; ou
• une mutation choisie parmi IVS14+1G>A, D949V, L155Stop et I560S est présente dans le gène de la Dihydropyrimidine Déshydrogénase et la concentration en uracile plasmatique est supérieure à 15 µg/L et le rapport des concentrations plasmatiques dihydrouracile/uracile est inférieur à 6.

2. Méthode selon la revendication 1, **caractérisée en ce que** ledit patient est atteint d'une maladie qui peut être traitée avec du 5-fluorouracile, de préférence un cancer, et **en ce qu'**elle comprend en outre l'évaluation de la dose de 5-fluorouracile adaptée audit patient.

3. Méthode selon la revendication 2, comprenant une étape supplémentaire de détermination de la dose de 5-fluorouracile à administrer au patient, dans laquelle la dose est déterminée à l'aide de l'algorithme suivant :
a) En cas
- d'absence de mutation choisie parmi IVS14+1G>A, D949V, L155Stop et I560S dans le gène de la Dihydropyrimidine Déshydrogénase et si la concentration en uracile plasmatique est inférieure à 15 µg/L, ou
- d'absence de mutation choisie parmi IVS14+1G>A, D949V, L155Stop et I560S dans le gène de la Dihydropyrimidine Déshydrogénase et si la concentration en uracile plasmatique est supérieure à 15 µg/l mais que le rapport des concentrations plasmatiques dihydrouracile/uracile est supérieur ou égal à 6,
la dose administrée de 5-fluorouracile est une dose standard
ii) Dans tous les autres cas, le rapport des concentrations plasmatiques dihydrouracile/uracile est mesuré pour déterminer la dose de 5-FU à administrer:
α) si ce rapport est supérieur à 6, la dose initiale de 5-fluorouracile est diminuée de 30% par rapport à la dose standard ;
ß) si ce rapport est compris entre 3 et 6, la dose initiale de 5-fluorouracile est réduite de 50% par rapport à la dose standard et la concentration plasmatique en 5-fluorouracile au cours des jours/heures suivant l'injection d'une dose de 5-fluorouracile est mesurée.
γ) si ce rapport est compris entre 1 et 3, la dose initiale de 5-fluorouracile est réduite de 70% par rapport à la dose standard et la concentration plasmatique en 5-fluorouracile au cours des jours/heures suivant l'injection d'une dose de 5-fluorouracile est mesurée
δ) si ce rapport est inférieur à 1, la dose initiale est réduite d'au moins 80% et la concentration plasmatique en 5-fluorouracile au cours des jours/heures suivant l'injection d'une dose de 5-fluorouracile est mesurée.

4. Méthode selon l'une quelconque des revendications 1 à 3, **caractérisée en ce que** le protocole de détermination à partir des cellules d'un échantillon provenant du sujet de la présence ou de l'absence d'une mutation dans le gène de la Dihydropyrimidine Déshydrogénase comporte les étapes suivantes:
- l'extraction de l'ADN génomique, ou l'extraction de l'ARN total et sa rétrotranscription en ADN complémentaire,
- l'amplification en parallèle ou en multiplex d'un ou plusieurs fragments du gène de la Dihydropyrimidine Déshydrogénase, et
- la détermination de la taille du produit d'amplification, où la taille du produit d'amplification indique la présence ou l'absence d'une mutation dans le gène de la Dihydropyrimidine Déshydrogénase.

5. Méthode selon l'une quelconque des revendications 1 à 3, **caractérisée en ce que** le protocole de détermination à partir des cellules d'un échantillon provenant du sujet de la présence ou de l'absence d'une mutation choisie parmi IVS14+1G>A, D949V, L155Stop et I560S dans le gène de la Dihydropyrimidine Déshydrogénase comporte les étapes suivantes :
- l'extraction de l'ADN génomique,
- l'amplification en parallèle ou en multiplex d'un ou plusieurs fragments du gène de la Dihydropyrimidine Déshydrogénase, et
- le séquençage des fragments amplifiés permettant l'identification de la présence ou l'absence d'une mutation dans le gène de la Dihydropyrimidine Déshydrogénase, ladite mutation étant choisie parmi IVS14+1G>A, D949V, L155Stop et I560S.

6. Méthode selon l'une quelconque des revendications 1 à 3, **caractérisée en ce que** le protocole de détermination de la présence ou de l'absence d'une mutation dans le gène de la Dihydropyrimidine Déshydrogénase est une combinaison des protocoles selon les revendications 4 et 5.

7. Méthode selon la revendication 5 ou 6, **caractérisée en ce que** la technique de séquençage utilisée est le pyroséquençage (ou mini-séquençage luminométrique).

8. Méthode selon l'une quelconque des revendications 4 à 7, **caractérisée en ce que** les amorces d'amplification utilisées sont choisies dans le groupe constitué de SEQ ID NO : 23, 24, 27, 28, 31, 32, 37 et 38.

9. Méthode selon l'une quelconque des revendications 5 à 7, **caractérisée en ce que** les amorces de séquence utilisées sont choisies dans le groupe constitué de SEQ ID NO : 53, 55, 57 et 60.

10. Méthode selon l'une quelconque des revendications 1 à 9, **caractérisée en ce que** la mesure de la concentration en uracile et/ou en dihydrouracile plasmatique est réalisée par chromatographie liquide haute performance avec détection UV, en utilisant une colonne possédant une phase stationnaire composée de particules de carbone sphériques totalement poreuses.

11. Méthode selon l'une quelconque des revendications 1 à 9, **caractérisée en ce que** ladite méthode est mise en oeuvre sur un ou plusieurs échantillons biologiques, dont au moins un prélèvement sanguin, d'un patient atteint d'une maladie, de préférence un cancer, qui peut être traitée avec du 5-fluorouracile.

## Patentansprüche

1. Verfahren zur *in vitro*-Diagnose der Prädisposition eines Patienten für die Entwicklung einer Toxizität nach einer Behandlung, die 5-Fluorouracil umfasst, wobei das Verfahren die Durchführung auf einem oder auf mehreren biologischen Proben des Patienten, darunter mindestens eine Blutentnahme, der folgenden Kombination umfasst:
- Bestimmung der Anwesenheit oder der Abwesenheit einer Mutation, ausgewählt aus IVS14+1G>A, D949V, L155Stop und I560S im Gen der Dihydropyrimidindehydrogenase, und
- Messen der Konzentration von plasmatischem Uracil und/oder des Verhältnisses der plasmatischen Dihydrouracil-/Uracilkonzentrationen,
wobei der Patient ein erhöhtes Risiko für die Entwicklung einer Toxizität nach einer Behandlung, die 5-Fluorouracil umfasst, aufweist, wenn:
- eine Mutation, ausgewählt aus IVS14+1G>A, D949V, L155Stop und I560S im Gen der Dihydropyrimidindehydrogenase anwesend ist und die Konzentration von plasmatischem Uracil höher als 15 µg/l ist; oder
- eine Mutation, ausgewählt aus IVS14+1G>A, D949V, L155Stop und I560S im Gen der Dihydropyrimidindehydrogenase anwesend ist und das Verhältnis der plasmatischen Dihydrouraci-/Uracil-Konzentrationen niedriger als 6 ist; oder
- eine Mutation, ausgewählt aus IVS14+1G>A, D949V, L155Stop und I560S im Gen der Dihydropyrimidindehydrogenase anwesend ist und die Konzentration von plasmatischem Uracil höher als 15 pg/' ist und das Verhältnis der plasmatische Dihydrouracil-/Uracil-Konzentrationen niedriger als 6 ist.

2. Verfahren nach Anspruch 1, **dadurch gekennzeichnet, dass** der Patient an einer Krankheit leidet, die mit 5-Fluorouracil behandelt werden kann, vorzugsweise einem Krebs, und dadurch, dass es außerdem die Bewertung der Dosis von 5-Fluorouracil, angepasst an den Patienten, umfasst.

3. Verfahren nach Anspruch 2, umfassend einen zusätzlichen Schritt des Bestimmens der Dosis von 5-Fluorouracil, die dem Patienten verabreicht werden muss, wobei die Dosis mit Hilfe des folgenden Algorithmus bestimmt wird:
a) im Fall der
- Abwesenheit einer Mutation, ausgewählt aus IVS14+1G>A, D949V, L155Stop und I560S im Gen der Dihydropyrimidindehydrogenase und wenn die Konzentration von plasmatischem Uracil kleiner als 15 µg/L ist, oder
- Abwesenheit einer Mutation, ausgewählt aus IVS14+1G>A, D949V, L155Stop und I560S im Gen der Dihydropyrimidindehydrogenase und wenn die Konzentration von plasmatischem Uracil höher als 15 µg/l ist, jedoch das Verhältnis der plasmatischen Dihydrouraci-/Uracil-Konzentrationen höher als oder gleich wie 6 ist;
ist die verabreichte Dosis von 5-Fluorouracil eine Standarddosis
ii) In allen anderen Fällen wird das Verhältnis der plasmatischen Dihydrouraci-/Uracil-Konzentrationen gemessen, um die Dosis von 5-FU zu bestimmen, die verabreicht werden muss:
α) wenn dieses Verhältnis höher als 6 ist, wird die anfängliche Dosis von 5-Fluorouracil um 30 % mit Bezug auf die Standarddosis verringert;
β) wenn dieses Verhältnis zwischen 3 und 6 liegt, wird die anfängliche Dosis von 5-Fluorouracil um 50 % mit Bezug auf die Standarddosis reduziert und die plasmatische Konzentration von 5-Fluorouracil im Laufe der Tage/Stunden nach der Injektion einer Dosis von 5-Fluorouracil gemessen.
γ) wenn dieses Verhältnis zwischen 1 und 3 liegt, wird die anfängliche Dosis von 5-Fluorouracil um 70 % mit Bezug auf die Standarddosis reduziert und die plasmatische Konzentration von 5-Fluorouracil im Laufe der Tage/Stunden nach der Injektion einer Dosis von 5-Fluorouracil gemessen
δ) wenn dieses Verhältnis kleiner als 1 ist, wird die anfängliche Dosis um mindestens 80 % reduziert und die plasmatische Konzentration von 5-Fluorouracil im Laufe der Tage/Stunden nach der Injektion einer Dosis von 5-Fluorouracil gemessen.

4. Verfahren nach irgendeinem der Ansprüche 1 bis 3, **dadurch gekennzeichnet, dass** das Protokoll der Bestimmung auf der Grundlage der Zellen einer Probe, die vom Subjekt stammt, der Anwesenheit oder der Abwesenheit einer Mutation im Gen der Dihydropyrimidindehydrogenase die folgenden Schritte umfasst:
- Extrahieren der genomischen DANN, oder Extrahieren der gesamten RNA und ihrer Retrotranskription an zusätzlicher DNA,
- Parallel- oder Multiplex-Amplifizieren von einem oder von mehreren Fragmenten des Gens der Dihydropyrimidindehydrogenase, und
- Bestimmen der Größe des Amplifizierungsprodukts, wobei die Größe des Amplifizierungsprodukts die Anwesenheit oder die Abwesenheit einer Mutation im Gen der Dihydropyrimidindehydrogenase angibt.

5. Verfahren nach irgendeinem der Ansprüche 1 bis 3, **dadurch gekennzeichnet, dass** das Protokoll der Bestimmung auf der Grundlage der Zellen einer Probe, die vom Subjekt stammt, der Anwesenheit oder der Abwesenheit einer Mutation, ausgewählt aus IVS14+1G>A, D949V, L155Stop und I560S, im Gen der Dihydropyrimidindehydrogenase die folgenden Schritte umfasst:
- Extrahieren der genomischen DNA,
- Parallel- oder Multiplex-Amplifizieren von einem oder von mehreren Fragmenten des Gens der Dihydropyrimidindehydrogenase, und
- Sequenzieren der amplifizierten Fragmente, die die Identifizierung der Anwesenheit oder der Abwesenheit einer Mutation im Gen der Dihydropyrimidindehydrogenase ermöglicht, wobei die Mutation ausgewählt ist aus IVS14+1G>A, D949V, L155Stop und I560S.

6. Verfahren nach irgendeinem der Ansprüche 1 bis 3, **dadurch gekennzeichnet, dass** das Protokoll der Identifizierung der Anwesenheit oder der Abwesenheit einer Mutation im Gen der Dihydropyrimidindehydrogenase eine Kombination der Protokolle gemäß den Ansprüchen 4 und 5 ist.

7. Verfahren nach Anspruch 5 oder 6, **dadurch gekennzeichnet, dass** die verwendete Technik der Sequenzierung die Pyrosequenzierung (oder luminometrische Minisequenzierung) ist.

8. Verfahren nach irgendeinem der Ansprüche 4 bis 7, **dadurch gekennzeichnet, dass** die verwendeten Amplifizierungsprimer ausgewählt sind aus der Gruppe, bestehend aus SEQ ID Nr.: 23, 24, 27, 28, 31, 32, 37 und 38.

9. Verfahren nach irgendeinem der Ansprüche 5 bis 7, **dadurch gekennzeichnet, dass** die verwendeten Sequenzprimer ausgewählt sind aus der Gruppe, bestehend aus SEQ ID Nr.: 53, 55, 57 und 60.

10. Verfahren nach irgendeinem der Ansprüche 1 bis 9, **dadurch gekennzeichnet, dass** die Messung der Konzentration von Uracil und/oder plasmatischem Dihydrouracil durch Hochleistungs-Füssigkeitschromatographie mit UV-Nachweis unter Verwendung einer Säule mit einer stationären Phase, zusammengesetzt aus vollständig porösen sphärischen Kohlenstoffpartikeln, durchgeführt wird.

11. Verfahren nach irgendeinem der Ansprüche 1 bis 9, **dadurch gekennzeichnet, dass** das Verfahren auf einem oder auf mehreren biologischen Proben durchgeführt wird, davon mindestens eine Blutentnahme, eines Patienten, der an einer Krankheit leidet, vorzugsweise einem Krebs, der mit 5-Fluorouracil behandelt werden kann.

## Claims

1. In vitro method for diagnosing the predisposition of a patient to develop toxicity following a treatment comprising 5-fluorouracil, said method comprising the carrying out on one or more biological samples of said patient, including at least one blood sample, of the combination:
- of the determining of the presence or of the absence of a mutation chosen from IVS14+1G>A, D949V, L155Stop and I560S in the gene of Dihydropyrimidine Deshydrogenase, and
- of the measuring of the concentration of uracil in plasma and/or the measuring of the concentration ratio of dihydrouracil/uracil in plasma,
with the patient having an increased risk of developing toxicity following a treatment comprising 5-fluorouracil if:
- a mutation chosen from IVS14+1G>A, D949V, L155Stop and I560S is present in the gene of Dihydropyrimidine Deshydrogenase and the concentration in uracil in plasma is greater than 15 pg/L; or
- a mutation chosen from IVS14+1G>A, D949V, L155Stop and I560S is present in the gene of Dihydropyrimidine Deshydrogenase and the concentration ratio of dihydrouracil/uracil in plasma is less than 6; or
- a mutation chosen from IVS14+1G>A, D949V, L155Stop and I560S is present in the gene of Dihydropyrimidine Deshydrogenase and the concentration of uracil in plasma is greater than 15 µg/L and the concentration ratio of dihydrouracil/uracil in plasma is less than 6.

2. Method according to claim 1, **characterised in that** said patient is afflicted with a disease that can be treated with 5-fluorouracil, preferably a cancer, and **in that** it further comprises the evaluating of the dose of 5-fluorouracil adapted to said patient.

3. Method according to claim 2, comprising an additional step of determining the dose of 5-fluorouracil to be administered to the patient, wherein the dose is determined using the following algorithm:
a) In case
- of absence of mutation chosen from IVS14+1G>A, D949V, L155Stop and I560S in the gene of Dihydropyrimidine Deshydrogenase and if the concentration of uracil in plasma is less than 15 µg/L, or
- of absence of mutation chosen from IVS14+1G>A, D949V, L155Stop and I560S in the gene of Dihydropyrimidine Deshydrogenase and if the concentration of uracil in plasma is greater than 15 µg/l but the concentration ratio of dihydrouracil/uracil in plasma is greater than or equal to 6,
the administered dose of 5-fluorouracil is a standard dose
ii) In all other cases, the concentration ratio of dihydrouracil/uracil in plasma is measured in order to determine the dose of 5-FU to be administered:
α) if this ratio is greater than 6, the initial dose of 5-fluorouracil is reduced 30% with respect to the standard dose;
β) if this ratio is between 3 and 6, the initial dose of 5-fluorouracil is reduced 50% with respect to the standard dose and the concentration in plasma of 5-fluorouracil during the days/hours following the injection of a dose of 5-fluorouracil is measured.
γ) if this ratio is between 1 and 3, the initial dose of 5-fluorouracil is reduced 70% with respect to the standard dose and the concentration in plasma of 5-fluorouracil during the days/hours following the injection of a dose of 5-fluorouracil is measured
δ) if this ratio is less than 1, the initial dose is reduced by at least 80% and the concentration in plasma of 5-fluorouracil during the days/hours following the injection of a dose of 5-fluorouracil is measured.

4. Method according to any of claims 1 to 3, **characterised in that** the protocol for determining using cells of a sample coming from the subject of the presence or of the absence of a mutation in the gene of Dihydropyrimidine Deshydrogenase comprises the following steps:
- the extracting of the genomic DNA, or the extraction of the total RNA and its retrotranscription in complementary DNA,
- the amplifying in parallel or in multiplex of one or more fragments of the gene of Dihydropyrimidine Deshydrogenase, and
- the determining of the size of the amplification product, where the size of the amplification product indicates the presence or the absence of a mutation in the gene of Dihydropyrimidine Deshydrogenase.

5. Method according to any of claims 1 to 3, **characterised in that** the protocol for determining using cells of a sample coming from the subject of the presence or of the absence of a mutation chosen from IVS14+1G>A, D949V, L155Stop and I560S in the gene of Dihydropyrimidine Deshydrogenase comprises the following steps:
- the extracting of the genomic DNA,
- the amplifying in parallel or in multiplex of one or several fragments of the gene of Dihydropyrimidine Deshydrogenase, and
- the sequencing of the amplified fragments allowing for the identification of the presence or of the absence of a mutation in the gene of Dihydropyrimidine Deshydrogenase, said mutation being chosen from IVS14+1G>A, D949V, L155Stop and I560S.

6. Method according to any of claims 1 to 3, **characterised in that** the protocol for determining the presence or the absence of a mutation in the gene of Dihydropyrimidine Deshydrogenase is a combination of the protocols according to claims 4 and 5.

7. Method according to claim 5 or 6, **characterised in that** the sequencing technique used is pyrosequencing (or luminometric mini-sequencing).

8. Method according to any of claims 4 to 7, **characterised in that** the amplification primers used are chosen in the group comprised of SEQ ID NO: 23, 24, 27, 28, 31, 32, 37 and 38.

9. Method according to any of claims 5 to 7, **characterised in that** the sequence primers used are chosen in the group comprised of SEQ ID NO: 53, 55, 57 and 60.

10. Method according to any of claims 1 to 9, **characterised in that** the measurement of the concentration in uracil and/or in dihydrouracil in plasma is taken via high-performance liquid chromatography with UV detection, by using a column having a stationary phase composed of totally porous spherical carbon particles.

11. Method according to any of claims 1 to 9, **characterised in that** said method is implemented on one or more biological samples, including at least one blood sample, of a patient afflicted with a disease, preferably a cancer, that can be treated with 5-fluorouracil.


### LISTE DE SEQUENCES

<110> Centre Régional de Lutte Contre le Cancer d'Angers
<120> Prévention des toxicités dues au 5-fluorouracile
<130> D23068
<160> 60
<170> PatentIn version 3.3
<210> 1
   <211> 20
   <212> ADN
   <213> Séquence artificielle
<220>
   <223> Amorce d'amplification
<400> 1
   atccaggact ctccccagat 20
<210> 2
   <211> 20
   <212> ADN
   <213> Séquence artificielle
<220>
   <223> Amorce d'amplification
<400> 2
   atccaggact ctccccagat 20
<210> 3
   <211> 22
   <212> ADN
   <213> Séquence artificielle
<220>
   <223> Amorce d'amplification
<400> 3
   gtctttagag tatcctggct tt 22
<210> 4
   <211> 25
   <212> ADN
   <213> Séquence artificielle
<220>
   <223> Amorce d'amplification
<400> 4
   tcttatcagg atttcttttc caatg 25
<210> 5
   <211> 19
   <212> ADN
   <213> Séquence artificielle
<220>
   <223> Amorce d'amplification
<400> 5
   cctggcttta aatctcgaa 19
<210> 6
   <211> 25
   <212> ADN
   <213> Séquence artificielle
<220>
   <223> Amorce d'amplification
<400> 6
   tcttatcagg atttcttttc caatg 25
<210> 7
   <211> 18
   <212> ADN
   <213> Séquence artificielle
<220>
   <223> Amorce d'amplification
<400> 7
   gagatgcctg aaatgtgc 18
<210> 8
   <211> 21
   <212> ADN
   <213> Séquence artificielle
<220>
   <223> Amorce d'amplification
<400> 8
   ttgtttgcaa tacttgtgat g 21
<210> 9
   <211> 19
   <212> ADN
   <213> Séquence artificielle
<220>
   <223> Amorce d'amplification
<400> 9
   tgtcagaaga gctgtccaa 19
<210> 10
   <211> 21
   <212> ADN
   <213> Séquence artificielle
<220>
   <223> Amorce d'amplification
<400> 10
   ttgtttgcaa tacttgtgat g 21
<210> 11
   <211> 20
   <212> ADN
   <213> Séquence artificielle
<220>
   <223> Amorce d'amplification
<400> 11
   ccacagatca gaaatccttc 20
<210> 12
   <211> 21
   <212> ADN
   <213> Séquence artificielle
<220>
   <223> Amorce d'amplification
<400> 12
   accaccaaca tattcttgtt t 21
<210> 13
   <211> 21
   <212> ADN
   <213> Séquence artificielle
<220>
   <223> Amorce d'amplification
<400> 13
   acttctgaaa ttcctcagtt c 21
<210> 14
   <211> 20
   <212> ADN
   <213> Séquence artificielle
<220>
   <223> Amorce d'amplification
<400> 14
   ctttacacca aggtccttca 20
<210> 15
   <211> 20
   <212> ADN
   <213> Séquence artificielle
<220>
   <223> Amorce d'amplification
<400> 15
   agataatttg cggtaaaagc 20
<210> 16
   <211> 20
   <212> ADN
   <213> Séquence artificielle
<220>
   <223> Amorce d'amplification
<400> 16
   agcagctttg tagccttttt 20
<210> 17
   <211> 18
   <212> ADN
   <213> Séquence artificielle
<220>
   <223> Amorce d'amplification
<400> 17
   cctgtcactc tccattgc 18
<210> 18
   <211> 20
   <212> ADN
   <213> Séquence artificielle
<220>
   <223> Amorce d'amplification
<400> 18
   tcctcaggga cagctcttat 20
<210> 19
   <211> 22
   <212> ADN
   <213> Séquence artificielle
<220>
   <223> Amorce d'amplification
<400> 19
   tggaacttgc taaggaagaa aa 22
<210> 20
   <211> 20
   <212> ADN
   <213> Séquence artificielle
<220>
   <223> Amorce d'amplification
<400> 20
   aaccaaaggc actgatgacc 20
<210> 21
   <211> 20
   <212> ADN
   <213> Séquence artificielle
<220>
   <223> Amorce d'amplification
<400> 21
   gtatttgcag gtggtgatgt 20
<210> 22
   <211> 23
   <212> ADN
   <213> Séquence artificielle
<220>
   <223> Amorce d'amplification
<400> 22
   ctgtacgtat ttgtgaatgt acc 23
<210> 23
   <211> 20
   <212> ADN
   <213> Séquence artificielle
<220>
   <223> Amorce d'amplification
<400> 23
   aatatggagc ttccgtttct 20
<210> 24
   <211> 20
   <212> ADN
   <213> Séquence artificielle
<220>
   <223> Amorce d'amplification
<400> 24
   gagagaaagt tttggtgagg 20
<210> 25
   <211> 20
   <212> ADN
   <213> Séquence artificielle
<220>
   <223> Amorce d'amplification
<400> 25
   ctttcatcag gacattgtga 20
<210> 26
   <211> 20
   <212> ADN
   <213> Séquence artificielle
<220>
   <223> Amorce d'amplification
<400> 26
   caccaactta tgccaattct 20
<210> 27
   <211> 20
   <212> ADN
   <213> Séquence artificielle
<220>
   <223> Amorce d'amplification
<400> 27
   atcagtgaga aaacggctgc 20
<210> 28
   <211> 23
   <212> ADN
   <213> Séquence artificielle
<220>
   <223> Amorce d'amplification
<400> 28
   taaacattca ccaacttatg cca 23
<210> 29
   <211> 18
   <212> ADN
   <213> Séquence artificielle
<220>
   <223> Amorce d'amplification
<400> 29
   tctgacctaa catgcttc 18
<210> 30
   <211> 20
   <212> ADN
   <213> Séquence artificielle
<220>
   <223> Amorce d'amplification
<400> 30
   aggccttttg gggataaaac 20
<210> 31
   <211> 21
   <212> ADN
   <213> Séquence artificielle
<220>
   <223> Amorce d'amplification
<400> 31
   aagcactgca gtaccttgga a 21
<210> 32
   <211> 24
   <212> ADN
   <213> Séquence artificielle
<220>
   <223> Amorce d'amplification
<400> 32
   tcatgtagca tttaccacag ttga 24
<210> 33
   <211> 20
   <212> ADN
   <213> Séquence artificielle
<220>
   <223> Amorce d'amplification
<400> 33
   ccctctattt ctgtttgcag 20
<210> 34
   <211> 20
   <212> ADN
   <213> Séquence artificielle
<220>
   <223> Amorce d'amplification
<400> 34
   agggtacgcc tctctttggt 20
<210> 35
   <211> 20
   <212> ADN
   <213> Séquence artificielle
<220>
   <223> Amorce d'amplification
<400> 35
   ccctctattt ctgtttgcag 20
<210> 36
   <211> 20
   <212> ADN
   <213> Séquence artificielle
<220>
   <223> Amorce d'amplification
<400> 36
   agggtacgcc tctctttggt 20
<210> 37
   <211> 20
   <212> ADN
   <213> Séquence artificielle
<220>
   <223> Amorce d'amplification
<400> 37
   ttatggagct gctaagatga 20
<210> 38
   <211> 23
   <212> ADN
   <213> Séquence artificielle
<220>
   <223> Amorce d'amplification
<400> 38
   atcatacata cctcagtagc aaa 23
<210> 39
   <211> 20
   <212> ADN
   <213> Séquence artificielle
<220>
   <223> Amorce de pyroséquençage
<400> 39
   atttgcaaag gtaagatact 20
<210> 40
   <211> 16
   <212> ADN
   <213> Séquence artificielle
<220>
   <223> Amorce de pyroséquençage
<400> 40
   cctggcttta aatcct 16
<210> 41
   <211> 18
   <212> ADN
   <213> Séquence artificielle
<220>
   <223> Amorce de pyroséquençage
<400> 41
   caaactcatg caactctg 18
<210> 42
   <211> 15
   <212> ADN
   <213> Séquence artificielle
<220>
   <223> Amorce de pyroséquençage
<400> 42
   aatgtgcaga tgccc 15
<210> 43
   <211> 18
   <212> ADN
   <213> Séquence artificielle
<220>
   <223> Amorce de pyroséquençage
<400> 43
   aactaatctt gatattaa 18
<210> 44
   <211> 16
   <212> ADN
   <213> Séquence artificielle
<220>
   <223> Amorce de pyroséquençage
<400> 44
   tgggcctgca agtata 16
<210> 45
   <211> 15
   <212> ADN
   <213> Séquence artificielle
<220>
   <223> Amorce de pyroséquençage
<400> 45
   ttggctcgat tgggg 15
<210> 46
   <211> 15
   <212> ADN
   <213> Séquence artificielle
<220>
   <223> Amorce de pyroséquençage
<400> 46
   gaaattcctc agttc 15
<210> 47
   <211> 20
   <212> ADN
   <213> Séquence artificielle
<220>
   <223> Amorce de pyroséquençage
<400> 47
   aatgaaatga ctcttagcac 20
<210> 48
   <211> 15
   <212> ADN
   <213> Séquence artificielle
<220>
   <223> Amorce de pyroséquençage
<400> 48
   tcgatacggg gagtc 15
<210> 49
   <211> 17
   <212> ADN
   <213> Séquence artificielle
<220>
   <223> Amorce de pyroséquençage
<400> 49
   gagacactgc ctttgac 17
<210> 50
   <211> 19
   <212> ADN
   <213> Séquence artificielle
<220>
   <223> Amorce de pyroséquençage
<400> 50
   tgctaaggaa gaaaagtgt 19
<210> 51
   <211> 20
   <212> ADN
   <213> Séquence artificielle
<220>
   <223> Amorce de pyroséquençage
<400> 51
   ggctaacact acagtggaat 20
<210> 52
   <211> 20
   <212> ADN
   <213> Séquence artificielle
<220>
   <223> Amorce de pyroséquençage
<400> 52
   tattgatctg gtggacatta 20
<210> 53
   <211> 19
   <212> ADN
   <213> Séquence artificielle
<220>
   <223> Amorce de pyroséquençage
<400> 53
   ccagccacca gcacatcaa 19
<210> 54
   <211> 20
   <212> ADN
   <213> Séquence artificielle
<220>
   <223> Amorce de pyroséquençage
<400> 54
   çcaactaatc ttgatattaa 20
<210> 55
   <211> 16
   <212> ADN
   <213> Séquence artificielle
<220>
   <223> Amorce de pyroséquençage
<400> 55
   aggctgactt tccaga 16
<210> 56
   <211> 17
   <212> ADN
   <213> Séquence artificielle
<220>
   <223> Amorce de pyroséquençage
<400> 56
   ttggacctta tctggaa 17
<210> 57
   <211> 17
   <212> ADN
   <213> Séquence artificielle
<220>
   <223> Amorce de pyroséquençage
<400> 57
   gcaagttgtg gctatga 17
<210> 58
   <211> 20
   <212> ADN
   <213> Séquence artificielle
<220>
   <223> Amorce de pyroséquençage
<400> 58
   acagtttgat ccagaaaccc 20
<210> 59
   <211> 20
   <212> ADN
   <213> Séquence artificielle
<220>
   <223> Amorce de pyroséquençage
<400> 59
   gctgtactct gtgtctcagt 20
<210> 60
   <211> 15
   <212> ADN
   <213> Séquence artificielle
<220>
   <223> Amorce de pyroséquençage
<400> 60
   cccattaata ttggt 15